# EUROPEAN PATENT APPLICATION

(11) **EP 4 242 231 A1**
(43) Date of publication of application: **13.09.2023**
(21) Application number: 21888626.5
(22) Date of filing: 04.11.2021
(51) Int. Cl.: C07K 16/28, A61K 39/395

(54) **ANTI-SIGLEC-15 ANTIBODY AND APPLICATION THEREOF IN PREPARING DRUG**

(30) Priority: 05.11.2020 CN 202011221548; 17.09.2021 CN 202111095390
(71) Applicant: Shanghai JMT-Bio Technology Co., Ltd., Shanghai 201318 (CN)
(72) Inventor: HE, Ke, Shanghai 201318 (CN); LIAO, Shihua, Shanghai 201318 (CN); FENG, Xu, Shanghai 201318 (CN); REN, Baolan, Shanghai 201318 (CN); DU, Yuhan, Shanghai 201318 (CN); CHEN, Yingjiao, Shanghai 201318 (CN); SONG, Liping, Shanghai 201318 (CN); FAN, Yi, Shanghai 201318 (CN)
(74) Representative: J A Kemp LLP
(86) International application number: PCT/CN2021/128759
(87) International publication number: WO 2022/095934

(57) **Abstract**

The present invention relates to the field of bio-pharmaceuticals and, in particular, to an anti-Siglec-15 antibody or an antigen binding fragment thereof, a nucleic acid sequence encoding the anti-Siglec-15 antibody of the present invention or an antigen binding fragment thereof, an expression vector containing the nucleic acid sequence, a host cell containing the expression vector, a composition or a bispecific antibody comprising the anti-Siglec-15 antibody of the present invention or an antigen binding fragment thereof, an immune conjugate comprising the anti-Siglec-15 antibody of the present invention or an antigen binding fragment thereof linked to a therapeutic agent, as well as use of the anti-Siglec-15 antibody of the present invention or an antigen binding fragment thereof in the preparation of a medicament for treatment of cancer. The anti-Siglec-15 antibody of the present invention or an antigen binding fragment thereof can bind to Siglec-15 and be useful for treatment of Siglec-15 relevant diseases.

## Description

### TECHNICAL FIELD

The present invention relates to the field of bio-pharmaceuticals and, in particular, to an anti-Siglec-15 antibody or an antigen binding fragment thereof, a nucleic acid sequence encoding the anti-Siglec-15 antibody of the present invention or an antigen binding fragment thereof, an expression vector containing the nucleic acid sequence, a host cell containing the expression vector, a composition or a bispecific antibody comprising the anti-Siglec-15 antibody of the present invention or an antigen binding fragment thereof, an immune conjugate comprising the anti-Siglec-15 antibody of the present invention or an antigen binding fragment thereof linked to a therapeutic agent, as well as use of the anti-Siglec-15 antibody of the present invention or an antigen binding fragment thereof in the preparation of a medicament for treatment of cancer.

### TECHNICAL BACKGROUND

Cancer has become an important killer of human health. Due to the aging population, industrialization, urbanization and lifestyle changes, the number of cancer cases and deaths in China continues to increase, and the cancer burden cannot be ignored. The development of cancer is the result of genetic and environmental interactions and is triggered by uncontrolled cell proliferation caused by the malignant transformation of a single cell gene in normal tissue. Treatment of cancer requires killing or removing all tumor cells with tolerable side effects. With the advancement of surgery, chemo- or radio-therapy and targeted therapy, the survival period of many tumor patients has been prolonged. Due to the metastasis of tumors and their heterogeneity, most patients develop drug resistance or relapse, and eventually die.

In the last decade, a new class of immunotherapy approaches has emerged, represented by immune checkpoint PD-1/PD-L1 inhibitors. These strategies mainly target tumor immune escape mechanisms. When the PD-1/PD-L1 pathway is activated in the tumor microenvironment, the antitumor immune response of effector T cells is suppressed. Treatments that block this pathway can effectively improve anti-tumor immune responses in many tumor types. With the launch of PD-1/PD-L1 antibody drugs, tumor immunotherapy has become an emerging therapeutic paradigm. Compared with chemoradiotherapy and targeted therapy, PD-1/PD-L1 antibody drugs can provide long-term remission for some patients. The immune system has great potential to specifically recognize and attack tumor cells without causing toxic side effects on normal tissue. In addition, the memory of the immune system prevents the recurrence of the tumor. However, PD-1/PD-L1 antibody drugs are only effective in 20%-30% of patients, and how to make more patients benefit from tumor immunotherapy is an urgent problem to be solved. This requires the search for new immune checkpoint therapeutic targets for drug development.

The sialic acid-binding immunoglobulin-like lectin (Siglec) family is a class of classical immunoglobulin-like lectins that play an important regulatory role in innate and adaptive immunity by identifying the sugar chain structure containing sialic acid and mediating cell-cell or pathogen-cell interactions. At present, 15 human-derived and 9 murine-derived Siglec molecules have been identified, and Siglec is specifically expressed on the surface of myeloid cells and immune cells. Siglec family proteins are type I transmembrane proteins, with immunoglobulin superfamily domains. Different family members have different numbers of "C2-set" immunoglobulin-like domains, which have high sequence and folding similarity to immunoglobulin constant region. The extracellular terminal immunoglobulin domain, also known as "V-set" domain, has a high similarity to the antibody variable region. Siglec family proteins also have a transmembrane region and an intracellular segment. Most Siglec intracellular segments contain immunoreceptor tyrosine-based inhibitory motifs (ITIMs) to exhibit immunosuppressive functions.

Siglec-15 is an evolutionarily highly conserved member of the Siglec family with two immunoglobulin-like domains in its extracellular region, the "C2-set" domain and the "V-set" domain. The transmembrane region contains a lysine residue (Lys274, human Siglec-15) that plays a key role in interacting with the 12 kDa signaling linker molecule DNAX-activated protein (DAP12). The interaction of Siglec-15 and DAP12 is occurred through ionic bonding in the transmembrane region. DAP12 has a very short extracellular domain (< 20 amino acids), a transmembrane region containing an aspartate residue (Asp50, human DAP12), and an intracellular region containing an ITAM (immunoreceptor tyrosine-based activating motif). DAP12 recruits SYK through the ITAM to achieve phosphorylation and signaling.

Early research on Siglec-15 focused on osteoclast-related diseases. Hiruma et al. reported inhibition of differentiation into osteoclasts from RAW264.7 mouse macrophage lines (often used as osteoclast precursor models), mouse bone marrow macrophages, and human osteoclast precursors by using polyclonal antibodies against Siglec-15. Ishida-Kitagawa et al. demonstrated that interactions between Siglec-15 and DAP12, SYK signaling, as well as interaction between Siglec-15 and sialylated ligands, are critical for osteoclast differentiation. These in vitro studies were quickly validated in transgenic mice. Hiruma et al. reported that Siglec15-deficient mice exhibited mild osteosclerosis (increased bone mass) in trabecular bones and decreased urinary deoxypyridineline (a systemic marker of bone resorption), indicating reduced osteoclast viability. Takahata's team verified the observations using an additional strain of Siglec15-deficient mice.

Recent studies found that Siglec-15 is expressed on tumor-associated macrophages and induced by M-CSF, a cytokine that induces selective activation/polarization of macrophages. Co-culture of sialyl-Tn+ tumor cell lines and M-CSF-induced human macrophages or Siglec-15+ myeloid cells enhances DAP12- and SYK-dependent production of TGF-β, a pleiotropic cytokine that promotes epithelial-mesenchymal transformation and metastasis of cancer cells, by myeloid cells. These results suggest that Siglec-15 may have an important role in the tumor microenvironment.

On March 5, 2019, *Natural Medicine* published the new findings of Professor Chen Lieping's team - Siglec-15 is a new immunosuppressive checkpoint molecule in the tumor immune microenvironment and can be a potential tumor immunotherapy target. Studies have found that Siglec-15 expressed on the surface of tumor cells or tumor-associated macrophages can interact with T cell surface receptors and inhibit the killing effects of T cells against tumors. Siglec-15 can induce the differentiation of monocytes into specific myelocytes, which inhibit T cell function and promote tumor growth. Furthermore, in vitro experiments found that tumor growth was reduced in Siglec-15 knock-out mice, and tumor growth in mice could be inhibited after treatment with Siglec-15 monoclonal antibodies. In addition, the expression ofPD-L1 and Siglec-15 is mutually exclusive, which indicates that Siglec-15 antibodies may be effective in patients who do not respond to anti-PD-1/PD-L1 immunotherapy. The above results suggest that Siglec-15 is another molecule involved in immune evasion in the tumor microenvironment and has the potential to be used as a therapeutic target.

There is currently an unmet need for oncology therapy in this field.

### SUMMARY OF THE INVENTION

In an aim to overcome the deficiency of lack of a medicament for treatment of siglec-15 relevant diseases, the present invention provides an anti-Siglec-15 antibody and uses thereof in the preparation of a medicament. The anti-Siglec-15 antibodies bind to Siglec-15 protein and exhibit many excellent properties, including, e.g., binding to Siglec-15 at high affinity. Further, the antibodies of the invention reversed the inhibition of PBMC proliferation induced by Siglec-15 and inhibited Siglec-15 mediated monocyte proliferation, leading to modulation of immune response. Thus, the present invention also relates to a method of modulating immune response using an anti-Siglec-15 antibody or an antigen binding fragment thereof. The present invention also provides use of an anti-Siglec-15 antibody or an antigen binding fragment thereof in the preparation of a medicament for treatment of a Siglec-15 relevant disease or of a tumor.

A first aspect of the invention provides an isolated antibody binding to Siglec-15 or an antigen binding fragment thereof. In some embodiments, provided is an anti-Siglec-15 antibody or an antigen binding fragment thereof, comprising one or more CDR (complementarity determining region) sequences selected from a group consisting of SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 81.

In further embodiments, provided is an isolated anti-Siglec-15 antibody or an antigen binding fragment thereof, comprising one or more heavy chain CDR sequences selected from a group consisting of SEQ ID NOs: 1, 2, 3, 4, 5, 11, 12, 13, 17, 18, 19, 20, 25, 26, 27, 28, 29, 35, 36, and 37, and/or, one or more light chain CDR sequences selected from a group consisting of SEQ ID NOs: 6, 7, 8, 9, 10, 14, 15, 16, 21, 22, 23, 24, 30, 31, 32, 33, 34, 38, 39, 40, and 81.

In some embodiments, provided is an anti-Siglec-15 antibody or an antigen binding fragment thereof, comprising a heavy chain variable region comprising HCDR1, HCDR2 and HCDR3 sequences, and/or a light chain variable region comprising LCDR1, LCDR2 and LCDR3 sequences, wherein:
(a) the HCDR1 sequence of the heavy chain variable region comprises an amino acid sequence selected from a group consisting of SEQ ID NOs: 1, 11, 17, 25, 35, and a conservatively modified variant each thereof; the HCDR2 sequence of the heavy chain variable region comprises an amino acid sequence selected from a group consisting of SEQ ID NOs: 2, 3, 4, 12, 18, 19, 26, 27, 28, 36, and a conservatively modified variant each thereof; and the HCDR3 sequence of the heavy chain variable region comprises an amino acid sequence selected from a group consisting of SEQ ID NOs: 5, 13, 20, 29, 37, and a conservatively modified variant each thereof;
(b) the LCDR1 sequence of the light chain variable region comprises an amino acid sequence selected from a group consisting of SEQ ID NOs: 6, 7, 14, 21, 30, 31, 38, 81, and a conservatively modified variant each thereof; the LCDR2 sequence of the light chain variable region comprises an amino acid sequence selected from a group consisting of SEQ ID NOs: 8, 9, 15, 22, 23, 32, 33, 39, and a conservatively modified variant each thereof; and the LCDR3 sequence of the light chain variable region comprises an amino acid sequence selected from a group consisting of SEQ ID NOs: 10, 16, 24, 34, 40, and a conservatively modified variant each thereof;
(c) the antibody or an antigen binding fragment thereof binds specifically to Siglec-15.

In one embodiment, provided is an isolated antibody binding to Siglec-15 or an antigen binding fragment thereof, comprising a heavy chain variable region and/or a light chain variable region, wherein the heavy chain variable region comprises a CDR sequence selected from a group consisting of SEQ ID NOs: 1, 2, 3, 4 and 5, and the light chain variable region comprises a CDR sequence selected from a group consisting of SEQ ID NOs: 6, 7, 8, 9, 10, and 81.

In another embodiment, provided is an isolated antibody binding to Siglec-15 or an antigen binding fragment thereof, comprising a heavy chain variable region and/or a light chain variable region, wherein the heavy chain variable region comprises a CDR sequence selected from a group consisting of SEQ ID NOs: 11, 12, and 13, and the light chain variable region comprises a CDR sequence selected from a group consisting of SEQ ID NOs: 14, 15, and 16.

In another embodiment, provided is an isolated antibody binding to Siglec-15 or an antigen binding fragment thereof, comprising a heavy chain variable region and/or a light chain variable region, wherein the heavy chain variable region comprises a CDR sequence selected from a group consisting of SEQ ID NOs: 17, 18, 19, and 20, and the light chain variable region comprises a CDR sequence selected from a group consisting of SEQ ID NOs: 21, 22, 23, and 24.

In another embodiment, provided is an isolated antibody binding to Siglec-15 or an antigen binding fragment thereof, comprising a heavy chain variable region and/or a light chain variable region, wherein the heavy chain variable region comprises a CDR sequence selected from a group consisting of SEQ ID NOs: 25, 26, 27, 28, and 29, and the light chain variable region comprises a CDR sequence selected from a group consisting of SEQ ID NOs: 30, 31, 32, 33, and 34.

In another embodiment, provided is an isolated antibody binding to Siglec-15 or an antigen binding fragment thereof, comprising a heavy chain variable region and/or a light chain variable region, wherein the heavy chain variable region comprises a CDR sequence selected from a group consisting of SEQ ID NOs: 35, 36, and 37, and the light chain variable region comprises a CDR sequence selected from a group consisting of SEQ ID NOs: 38, 39, and 40.

In one embodiment, provided is an isolated antibody binding to Siglec-15 or an antigen binding fragment thereof, comprising a heavy chain variable region comprising HCDR1, HCDR2 and HCDR3, and/or a light chain variable region comprising LCDR1, LCDR2 and LCDR3, wherein the HCDR1 sequence of the heavy chain variable region is shown in SEQ ID NO: 1, the HCDR2 sequence of the heavy chain variable region is selected from a group consisting of SEQ ID NOs: 2, 3, and 4, the HCDR3 sequence of the heavy chain variable region is shown in SEQ ID NO: 5, the LCDR1 sequence of the light chain variable region is selected from a group consisting of SEQ ID NOs: 6, 7, and 81, the LCDR2 sequence of the light chain variable region is selected from a group consisting of SEQ ID NOs: 8 and 9, and the LCDR3 sequence of the light chain variable region is shown in SEQ ID NO: 10.

In another embodiment, provided is an isolated antibody binding to Siglec-15 or an antigen binding fragment thereof, comprising a heavy chain variable region comprising HCDR1, HCDR2 and HCDR3, and/or a light chain variable region comprising LCDR1, LCDR2 and LCDR3, wherein the HCDR1 sequence of the heavy chain variable region is shown in SEQ ID NO: 11, the HCDR2 sequence of the heavy chain variable region is shown in SEQ ID NO: 12, the HCDR3 sequence of the heavy chain variable region is shown in SEQ ID NO: 13, the LCDR1 sequence of the light chain variable region is shown in SEQ ID NO: 14, the LCDR2 sequence of the light chain variable region is shown in SEQ ID NO: 15, and the LCDR3 sequence of the light chain variable region is shown in SEQ ID NO: 16.

In another embodiment, provided is an isolated antibody binding to Siglec-15 or an antigen binding fragment thereof, comprising a heavy chain variable region comprising HCDR1, HCDR2 and HCDR3, and/or a light chain variable region comprising LCDR1, LCDR2 and LCDR3, wherein the HCDR1 sequence of the heavy chain variable region is shown in SEQ ID NO: 17, the HCDR2 sequence of the heavy chain variable region is selected from a group consisting of SEQ ID NOs: 18 and 19, the HCDR3 sequence of the heavy chain variable region is shown in SEQ ID NO: 20, the LCDR1 sequence of the light chain variable region is shown in SEQ ID NO: 21, the LCDR2 sequence of the light chain variable region is selected from a group consisting of SEQ ID NOs: 22 and 23, and the LCDR3 sequence of the light chain variable region is shown in SEQ ID NO: 24.

In another embodiment, provided is an isolated antibody binding to Siglec-15 or an antigen binding fragment thereof, comprising a heavy chain variable region comprising HCDR1, HCDR2 and HCDR3, and/or a light chain variable region comprising LCDR1, LCDR2 and LCDR3, wherein the HCDR1 sequence of the heavy chain variable region is shown in SEQ ID NO: 25, the HCDR2 sequence of the heavy chain variable region is selected from a group consisting of SEQ ID NOs: 26, 27, and 28, the HCDR3 sequence of the heavy chain variable region is shown in SEQ ID NO: 29, the LCDR1 sequence of the light chain variable region is selected from a group consisting of SEQ ID NOs: 30 and 31, the LCDR2 sequence of the light chain variable region is selected from a group consisting of SEQ ID NOs: 32 and 33, and the LCDR3 sequence of the light chain variable region is shown in SEQ ID NO: 34.

In another embodiment, provided is an isolated antibody binding to Siglec-15 or an antigen binding fragment thereof, comprising a heavy chain variable region comprising HCDR1, HCDR2 and HCDR3, and/or a light chain variable region comprising LCDR1, LCDR2 and LCDR3, wherein the HCDR1 sequence of the heavy chain variable region is shown in SEQ ID NO: 35, the HCDR2 sequence of the heavy chain variable region is shown in SEQ ID NO: 36, the HCDR3 sequence of the heavy chain variable region is shown in SEQ ID NO: 37, the LCDR1 sequence of the light chain variable region is shown in SEQ ID NO: 38, the LCDR2 sequence of the light chain variable region is shown in SEQ ID NO: 39, and the LCDR3 sequence of the light chain variable region is shown in SEQ ID NO: 40.

The antibody of the invention or an antigen binding fragment thereof may be a murine antibody, a chimeric antibody, a humanized antibody, a human antibody, or a fragment each thereof, and preferably, a human antibody, a humanized antibody, or a fragment each thereof.

In one embodiment, provided is an isolated antibody binding to Siglec-15 or an antigen binding fragment thereof, comprising a heavy chain variable region as shown in SEQ ID NO: 41 or a variant thereof; and/or a light chain variable region comprising SEQ ID NO: 42 or a variant thereof.

In one embodiment, provided is an isolated antibody binding to Siglec-15 or an antigen binding fragment thereof, comprising a heavy chain variable region as shown in SEQ ID NO: 43 or a variant thereof; and/or a light chain variable region comprising SEQ ID NO: 44 or a variant thereof.

In one embodiment, provided is an isolated antibody binding to Siglec-15 or an antigen binding fragment thereof, comprising a heavy chain variable region as shown in SEQ ID NO: 45 or a variant thereof; and/or a light chain variable region comprising SEQ ID NO: 46 or a variant thereof.

In one embodiment, provided is an isolated antibody binding to Siglec-15 or an antigen binding fragment thereof, comprising a heavy chain variable region as shown in SEQ ID NO: 47 or a variant thereof; and/or a light chain variable region comprising SEQ ID NO: 48 or a variant thereof.

In one embodiment, provided is an isolated antibody binding to Siglec-15 or an antigen binding fragment thereof, comprising a heavy chain variable region as shown in SEQ ID NO: 49 or a variant thereof; and/or a light chain variable region comprising SEQ ID NO: 50 or a variant thereof.

In one embodiment, provided is an isolated antibody binding to Siglec-15 or an antigen binding fragment thereof, comprising: a heavy chain variable region comprising an amino acid sequence having at least 90%, 95%, 96%, 97%, 98%, or 99% homology to SEQ ID NO: 41; and/or a light chain variable region comprising an amino acid sequence having at least 90%, 95%, 96%, 97%, 98%, or 99% homology to SEQ ID NO: 42.

In one embodiment, provided is an isolated antibody binding to Siglec-15 or an antigen binding fragment thereof, comprising: a heavy chain variable region comprising an amino acid sequence having at least 90%, 95%, 96%, 97%, 98%, or 99% homology to SEQ ID NO: 43; and/or a light chain variable region comprising an amino acid sequence having at least 90%, 95%, 96%, 97%, 98%, or 99% homology to SEQ ID NO: 44.

In one embodiment, provided is an isolated antibody binding to Siglec-15 or an antigen binding fragment thereof, comprising: a heavy chain variable region comprising an amino acid sequence having at least 90%, 95%, 96%, 97%, 98%, or 99% homology to SEQ ID NO: 45; and/or a light chain variable region comprising an amino acid sequence having at least 90%, 95%, 96%, 97%, 98%, or 99% homology to SEQ ID NO: 46.

In one embodiment, provided is an isolated antibody binding to Siglec-15 or an antigen binding fragment thereof, comprising: a heavy chain variable region comprising an amino acid sequence having at least 90%, 95%, 96%, 97%, 98%, or 99% homology to SEQ ID NO: 47; and/or a light chain variable region comprising an amino acid sequence having at least 90%, 95%, 96%, 97%, 98%, or 99% homology to SEQ ID NO: 48.

In one embodiment, provided is an isolated antibody binding to Siglec-15 or an antigen binding fragment thereof, comprising: a heavy chain variable region comprising an amino acid sequence having at least 90%, 95%, 96%, 97%, 98%, or 99% homology to SEQ ID NO: 49; and/or a light chain variable region comprising an amino acid sequence having at least 90%, 95%, 96%, 97%, 98%, or 99% homology to SEQ ID NO: 50.

In a second aspect, provided is an isolated humanized antibody binding to Siglec-15 or an antigen binding fragment thereof, comprising a heavy chain variable region of a sequence shown in SEQ ID NO: 41 with one or more mutations introduced at a position selected from a group consisting of M3, K19, S40, E42, R44, P61, T65, S77, T78, S84, S88, M93, V97, P117 and/or S120, and/or a light chain variable region of a sequence shown in SEQ ID NO: 42 with one or more mutations introduced at a position selected from a group consisting of V3, L4, A9, A12, V13, L15, Q17, A19, S22, K24, D32, Q46, P47, E59, I62, A64, N78, H80, P81, V82, E83, E84, A87, A104 and/or L110.

In a second aspect, provided is an isolated humanized antibody binding to Siglec-15 or an antigen binding fragment thereof, comprising a heavy chain variable region of a sequence shown in SEQ ID NO: 41 with one or more mutations introduced at a position selected from a group consisting of M3Q, K19R, S40A, E42G, R44G, P61A, T65K, S77N, T78S, S84N, S88A, M93V, V97A, P117Q and/or S120T, and/or a light chain variable region of a sequence shown in SEQ ID NO: 42 with one or more mutations introduced at a position selected from a group consisting of V3Q, L4M, A9S, A12S, V13A, L15V, Q17D, A19V, S22T, K24R, D32E, Q46K, P47A, E59Q, I62V, A64S, N78T, H80S, P81S, V82L, E83Q, E84P, A87F, A104Q and/or L110I.

In one embodiment, provided is an isolated humanized antibody binding to Siglec-15 or an antigen binding fragment thereof, comprising a heavy chain variable region selected from a group consisting of SEQ ID NOs: 51, 53, 55, and 57, and/or a light chain variable region selected from a group consisting of SEQ ID NOs: 52, 54, 56, 58, and 82.

In one embodiment, provided is an isolated humanized antibody binding to Siglec-15 or an antigen binding fragment thereof, comprising a heavy chain variable region comprising a sequence shown in SEQ ID NO: 51 or a variant thereof, and a light chain variable region comprising a sequence shown in SEQ ID NO: 52 or a variant thereof.

In one embodiment, provided is an isolated humanized antibody binding to Siglec-15 or an antigen binding fragment thereof, comprising a heavy chain variable region comprising a sequence shown in SEQ ID NO: 51 or a variant thereof, and a light chain variable region comprising a sequence shown in SEQ ID NO: 52 or a variant thereof.

In one embodiment, provided is an isolated humanized antibody binding to Siglec-15 or an antigen binding fragment thereof, comprising a heavy chain variable region comprising a sequence shown in SEQ ID NO: 53 or a variant thereof, and a light chain variable region comprising a sequence shown in SEQ ID NO: 54 or a variant thereof.

In one embodiment, provided is an isolated humanized antibody binding to Siglec-15 or an antigen binding fragment thereof, comprising a heavy chain variable region comprising a sequence shown in SEQ ID NO: 55 or a variant thereof, and a light chain variable region comprising a sequence shown in SEQ ID NO: 56 or a variant thereof.

In one embodiment, provided is an isolated humanized antibody binding to Siglec-15 or an antigen binding fragment thereof, comprising a heavy chain variable region comprising a sequence shown in SEQ ID NO: 57 or a variant thereof, and a light chain variable region comprising a sequence shown in SEQ ID NO: 58 or a variant thereof.

In one embodiment, provided is an isolated humanized antibody binding to Siglec-15 or an antigen binding fragment thereof, comprising a heavy chain variable region comprising a sequence shown in SEQ ID NO: 53 or a variant thereof, and a light chain variable region comprising a sequence shown in SEQ ID NO: 56 or a variant thereof.

In one embodiment, provided is an isolated humanized antibody binding to Siglec-15 or an antigen binding fragment thereof, comprising a heavy chain variable region comprising a sequence shown in SEQ ID NO: 53 or a variant thereof, and a light chain variable region comprising a sequence shown in SEQ ID NO: 58 or a variant thereof.

In one embodiment, provided is an isolated humanized antibody binding to Siglec-15 or an antigen binding fragment thereof, comprising a heavy chain variable region comprising a sequence shown in SEQ ID NO: 53 or a variant thereof, and a light chain variable region comprising a sequence shown in SEQ ID NO: 82 or a variant thereof.

A third aspect of the invention provides an isolated humanized antibody binding to Siglec-15 or an antigen binding fragment thereof, comprising a heavy chain variable region of a sequence shown in SEQ ID NO: 45 with one or more mutations introduced at a position selected from a group consisting of E1, Q5, L11, V12, R13, L20, T23, K38, R40, T41, E42, A61, P62, K67, A68, A72, S76, N77, L81, H82, T85, T87, V97, and/or S113, and/or a light chain variable region of a sequence shown in SEQ ID NO: 46 with one or more mutations introduced at a position selected from a group consisting of S8, F12, A18, Y36, L41, P43, K45, V47, T58, D74, G78, N81, I82, P84, and/or V89.

In one embodiment, provided is an isolated humanized antibody binding to Siglec-15 or an antigen binding fragment thereof, comprising a heavy chain variable region of a sequence shown in SEQ ID NO: 45 with one or more mutations introduced at a position selected from a group consisting of E1Q, Q5V, L11V, V12K, R13K, L20V, T23K, K38R, R40A, T41P, E42G, A61S, P62Q, K67R, A68V, A72R, S76T or S76A, N77S, L81M, H82E, T85S, T87R, V97A, and/or S113T, and/or a light chain variable region of a sequence shown in SEQ ID NO: 46 with one or more mutations introduced at a position selected from a group consisting of S8P, F12A, A18V, Y36H, L41E, P43G, K45R, V47L, T58K, D74E, G78T, N81S, I82L, P84S, and/or V89D.

In one embodiment, provided is an isolated humanized antibody binding to Siglec-15 or an antigen binding fragment thereof, comprising a heavy chain variable region selected from a group consisting of SEQ ID NOs: 59, 61, 63, and 65, and/or a light chain variable region selected from a group consisting of SEQ ID NOs: 60, 62, 64, and 66.

In one embodiment, provided is an isolated humanized antibody binding to Siglec-15 or an antigen binding fragment thereof, comprising a heavy chain variable region comprising a sequence shown in SEQ ID NO: 59 or a variant thereof, and a light chain variable region comprising a sequence shown in SEQ ID NO: 60 or a variant thereof.

In one embodiment, provided is an isolated humanized antibody binding to Siglec-15 or an antigen binding fragment thereof, comprising a heavy chain variable region comprising a sequence shown in SEQ ID NO: 61 or a variant thereof, and a light chain variable region comprising a sequence shown in SEQ ID NO: 62 or a variant thereof.

In one embodiment, provided is an isolated humanized antibody binding to Siglec-15 or an antigen binding fragment thereof, comprising a heavy chain variable region comprising a sequence shown in SEQ ID NO: 63 or a variant thereof, and a light chain variable region comprising a sequence shown in SEQ ID NO: 64 or a variant thereof.

A fourth aspect of the invention provides an isolated humanized antibody binding to Siglec-15 or an antigen binding fragment thereof, comprising a heavy chain variable region of a sequence shown in SEQ ID NO: 47 with one or more mutations introduced at a position selected from a group consisting of E1, Q5, P9, V10, L11, V12, M20, E23, K38, S40, K43, S44, N61, K67, A68, L70, V72, S76, N84, T87, S91, and/or S119, and/or a light chain variable region of a sequence shown in SEQ ID NO: 48 with one or more mutations introduced at a position selected from a group consisting of V3, L4, A9, A12, V13, L15, Q17, A19, S22, R43, R46, P47, E59, I62, A64, R72, D80, P81, V82, E83, A84, D85, V87, A104, and/or L110.

In one embodiment, provided is an isolated humanized antibody binding to Siglec-15 or an antigen binding fragment thereof, comprising a heavy chain variable region of a sequence shown in SEQ ID NO: 47 with one or more mutations introduced at a position selected from a group consisting of E1Q, Q5V, P9A, V10E, L11V, V12K, M20V, E23K, K38R, S40A, K43Q, S44G, N61A, K67R, A68V, L70M, V72R, S76T, N84S, T87R, S91T, and/or S119T, and/or a light chain variable region of a sequence shown in SEQ ID NO: 48 with one or more mutations introduced at a position selected from a group consisting of V3Q, L4M, A9S, A12S, V13A, L15V, Q17D, A19V, S22T, R43K, R46K, P47A, E59Q, I62V, A64S, R72G, D80S, P81S, V82L, E83Q, A84P, D85E, V87F, A104Q, and/or L110I.

In one embodiment, provided is an isolated humanized antibody binding to Siglec-15 or an antigen binding fragment thereof, comprising a heavy chain variable region selected from a group consisting of SEQ ID NOs: 67, 69, 71, and 73, and/or a light chain variable region selected from a group consisting of SEQ ID NOs: 68, 70, 72, and 74.

In one embodiment, provided is an isolated humanized antibody binding to Siglec-15 or an antigen binding fragment thereof, comprising a heavy chain variable region comprising a sequence shown in SEQ ID NO: 67 or a variant thereof, and a light chain variable region comprising a sequence shown in SEQ ID NO: 68 or a variant thereof.

In one embodiment, provided is an isolated humanized antibody binding to Siglec-15 or an antigen binding fragment thereof, comprising a heavy chain variable region comprising a sequence shown in SEQ ID NO: 69 or a variant thereof, and a light chain variable region comprising a sequence shown in SEQ ID NO: 70 or a variant thereof.

In one embodiment, provided is an isolated humanized antibody binding to Siglec-15 or an antigen binding fragment thereof, comprising a heavy chain variable region comprising a sequence shown in SEQ ID NO: 71 or a variant thereof, and a light chain variable region comprising a sequence shown in SEQ ID NO: 72 or a variant thereof.

In any of the embodiments described above, the variant of the antibody of the invention or an antigen binding fragment thereof may comprise one, two or three conservative amino acid substitutions.

In any of the embodiments described above, the "Capital Letter + number" indicates the amino acid residue at the designated position number, and the "Capital Letter 1 + number + Capital Letter 2" indicates the amino acid residue at the designated position number is substituted by another amino acid residue. For example, "E1" represents the amino acid residue at position number 1 is glutamic acid; "E1Q" represents the glutamic acid at position 1 is substituted by glutamine, "Q5" represents the amino acid residue at position number 5 is glutamine, and "QSV" represents the glutamic acid at position 5 is substituted by valine.

In any of the embodiments described above, the antibody of the invention or an antigen binding fragment thereof may comprise: a human heavy chain constant region or a variant thereof, wherein the variant comprises up to 20 conservative amino acid substitutions; and/or a human light chain constant region or a variant thereof, wherein the variant comprises up to 20 conservative amino acid substitutions. In some embodiments, the variant may comprise up to 10 conservative amino acid substitutions. In some embodiments, the variant may comprise up to 5 conservative amino acid substitutions. In some embodiments, the variant may comprise up to 3 conservative amino acid substitutions. In any of the embodiments described above, the human heavy chain constant region or a variant thereof may be IgG1, IgG2, IgG3, IgG4, IgA, IgE, IgM or IgD constant region or a variant each thereof, preferably, IgG1 or IgG4 constant region or a variant each thereof.

The numbering of amino acid residues in the antibody of the invention or an antigen binding fragment thereof is in accordance with Kabat EU index numbering system.

In any of the embodiments described above, the antibody of the invention or an antigen binding fragment thereof may bind to Siglec-15 at a KD of 930 pM or less. In another embodiment, the antibody or an antigen binding fragment thereof may bind to Siglec-15 at a KD of 614 pM or less. In another embodiment, the antibody or an antigen binding fragment thereof may bind to Siglec-15 at a KD of 325 pM or less. In another embodiment, the antibody or an antigen binding fragment thereof may bind to Siglec-15 at a KD of 202 pM or less. In another embodiment, the antibody or an antigen binding fragment thereof may bind to Siglec-15 at a KD of 109 pM or less. In another embodiment, the antibody or an antigen binding fragment thereof may bind to Siglec-15 at a KD of 85.6 pM or less. In another embodiment, the antibody or an antigen binding fragment thereof may bind to Siglec-15 at a KD of 80.6 pM or less. In another embodiment, the antibody or an antigen binding fragment thereof may bind to Siglec-15 at a KD of 72.4 pM or less. In another embodiment, the antibody or an antigen binding fragment thereof may bind to Siglec-15 at a KD of 68.3 pM or less.

The KD value can be determined by any effective methods. In a preferable embodiment, the dissociation constant is determined by biolayer interferometry, such as ForteBio Octet system used in Example 2. In additional embodiments, the dissociation constant can be determined by surface plasmon resonance (e.g., Biacore) or Kinexa.

In some embodiments, the antigen binding fragment of the antibody of the invention includes, but is not limited to, antibody fragments like Fab, Fab', F(ab')₂, Fv, scFv, or sdFv (disulfide linked Fv).

In the present invention, fragment Fab refers to a monovalent fragment consisting of VL, VH, CL, and CH1 domains; fragment F(ab')₂ refers to a bivalent fragment comprising two Fab fragments linked via a disulfide bridge in the hinge region; fragment Fd consists of VH and CH1 domains; fragment Fv consists of VL and VH domains from a single arm of the antibody; and fragment scFv is a monovalent molecule formed by paring of VL and VH domains.

In some embodiments, the anti-Siglec-15 antibody of the invention or an antigen binding fragment thereof may bind to human Siglec-15. In further embodiments, the anti-Siglec-15 antibody of the invention or an antigen binding fragment thereof may bind to monkey Siglec-15. In further embodiments, the anti-Siglec-15 antibody of the invention or an antigen binding fragment thereof may bind to murine Siglec-15.

In some embodiments, the anti-Siglec-15 antibodies of the invention or antigen binding fragments thereof bind to the same epitope or different epitopes of Siglec-15.

In some embodiments, the fragment of the antibody of the invention is a bispecific antibody.

A fifth aspect of the invention provides a bispecific antibody comprising any of the antibody binding to Siglec-15 or an antigen binding fragment thereof as described above.

A sixth aspect of the invention provides a nucleic acid sequence encoding an anti-Siglec-15 antibody of the invention or an antigen binding fragment thereof.

A seventh aspect of the invention provides an expression vector comprising a nucleic acid sequence encoding an anti-Siglec-15 antibody of the invention or an antigen binding fragment thereof.

An eighth aspect of the invention provides a host cell comprising said expression vector.

Further, a ninth aspect of the invention provides a method for preparing the antibody of the invention or an antigen binding fragment thereof, comprising (a) culturing a host cell containing a nucleic acid encoding the antibody of the invention or an antigen binding fragment thereof in a culture medium in a condition enabling the expression of the nucleic acid, thereby producing a polypeptide comprising a light chain variable region and a heavy chain variable region; and (b) recovering the polypeptide from the host cell or the culture medium.

In a tenth aspect of the invention, provided is a composition comprising an antibody of the invention or an antigen binding fragment thereof, and a pharmaceutically acceptable carrier or diluent.

In an eleventh aspect of the invention, provided is an immune conjugate comprising an antibody of the invention or an antigen binding fragment thereof, linked to a therapeutic agent, such as a cytotoxin or a radiotoxin. Non-limiting examples of cytotoxins include antimetabolites, alkylating agents, antibiotics, and antimitotic agents, such as taxol, cytochalasin B, mitomycin, etoposide, and vincristine. In a twelfth aspect of the invention, provided is use of the anti-Siglec-15 antibody or an antigen binding fragment thereof of the first aspect, the composition of the tenth aspect, the immune conjugate of the eleventh aspect, or the bispecific antibody of the fifth aspect in the preparation of a medicament for treatment of a Siglec-15 relevant disease.

Alternatively, provided is the anti-Siglec-15 antibody or an antigen binding fragment thereof of the first aspect, the composition of the tenth aspect, the immune conjugate of the eleventh aspect, or the bispecific antibody of the fifth aspect for use in the treatment of a Siglec-15 relevant disease.

Alternatively, provided is a method for treatment of a Siglec-15 relevant disease, comprising administering to a subject in need thereof the anti-Siglec-15 antibody or an antigen binding fragment thereof of the first aspect, the composition of the tenth aspect, the immune conjugate of the eleventh aspect, or the bispecific antibody of the fifth aspect.

The Siglec-15 relevant disease in the present invention includes, but is not limited to, a cancer or a bone disease. The cancer or the bone disease can be a Siglec-15 positive cancer or bone disease;

The cancer includes, but is not limited to, leukemia, melanoma, head and neck cancer, glioblastoma, breast cancer, ovarian cancer, gastric cancer, urothelium carcinoma, gastroesophageal carcinoma, esophagus cancer, endometrial cancer, colon cancer, liver cancer, lung cancer or bladder cancer, wherein the leukemia includes acute lymphocytic leukemia (ALL), acute myelogenous leukemia (AML), chronic lymphocytic leukemia (CLL), and chronic myelogenous leukemia (CML);

The bone disease includes, but is not limited to, osteoporosis, bone loss, osteoarthritis, osteomalacia, and the like.

Further provided herein is use of the anti-Siglec-15 antibody of the invention or an antigen binding fragment thereof in the preparation of a medicament for reversing inhibition of PBMC proliferation mediated by Siglec-15-hFc.

Further provided herein is use of the anti-Siglec-15 antibody of the invention or an antigen binding fragment thereof in the preparation of a medicament for inhibiting monocyte proliferation mediated by Siglec-15 protein.

Further provided herein is use of the anti-Siglec-15 antibody of the invention or an antigen binding fragment thereof in the preparation of a medicament for inhibiting monocyte survival mediated by Siglec-15 protein.

Further provided herein is use of the anti-Siglec-15 antibody of the invention or an antigen binding fragment thereof in the preparation of a medicament for modulating immune response.

A thirteenth aspect of the invention provides use of the anti-Siglec-15 antibody or an antigen binding fragment thereof of the first aspect, the composition of the tenth aspect, the immune conjugate of the eleventh aspect, or the bispecific antibody of the fifth aspect in the preparation of a medicament for inhibiting tumor cell growth.

Alternatively, provided is the anti-Siglec-15 antibody or an antigen binding fragment thereof of the first aspect, the composition of the tenth aspect, the immune conjugate of the eleventh aspect, or the bispecific antibody of the fifth aspect for use in inhibiting tumor cell growth.

Alternatively, provided is a method for inhibiting tumor cell growth, comprising administering to a subject in need thereof the anti-Siglec-15 antibody or an antigen binding fragment thereof of the first aspect, the composition of the tenth aspect, the immune conjugate of the eleventh aspect, or the bispecific antibody of the fifth aspect.

In some embodiments, the anti-Siglec-15 antibody or an antigen binding fragment thereof may be combined with a second therapeutic agent or therapy.

In one embodiment, the treatment with the anti-Siglec-15 antibody or an antigen binding fragment thereof may be combined with a chemotherapy.

In one embodiment, the treatment with the anti-Siglec-15 antibody or an antigen binding fragment thereof may be combined with a radiotherapy.

In some embodiments, the anti-Siglec-15 antibody or an antigen binding fragment thereof may be combined with another therapeutic antibody used for cancer treatment.

The advantages and effects of the present invention reside in provisions of novel antibodies or antigen binding fragments thereof that have high affinity with Siglec-15, preparation methods thereof, compositions, immune conjugates, or bispecific antibodies containing the antibodies or the fragments. The anti-Siglec-15 antibodies and antigen binding fragments thereof provided herein can be used for modulating immune response and for treating tumors.

### Brief Description of the Drawings

Figure 1 shows the binding curves and EC50 values of anti-Siglec-15 chimeric antibodies to 293T-hu-S15.
Figure 2A shows curve and EC50 value of chimeric antibody 21F6C6 reversing inhibition of PBMC proliferation induced by Siglec-15 protein.
Figure 2B shows curve and EC50 value of chimeric antibody 75E1F2 reversing PBMC proliferation inhibition induced by Siglec-15 protein.
Figure 2C shows cure and EC50 value of chimeric antibody 174C11C9 reversing PBMC proliferation inhibition induced by Siglec-15 protein.
Figure 2D shows curve and EC50 value of chimeric antibody 124B11F6 reversing PBMC proliferation inhibition induced by Siglec-15 protein.
Figure 2E shows curve and EC50 value of chimeric antibody 255C12B10 reversing inhibition of PBMC proliferation induced by Siglec-15 protein.
Figure 3 shows anti-Siglec-15 chimeric antibodies inhibited Siglec-15 protein mediated monocyte proliferation.
Figure 4A depicts humanization protocol of the heavy chain variable region of chimeric antibody 21F6C6.
Figure 4B depicts humanization protocol of the light chain variable region of chimeric antibody 21F6C6.
Figure 4C depicts humanization protocol of the heavy chain variable region of chimeric antibody 124B11F6.
Figure 4D depicts humanization protocol of the light chain variable region of chimeric antibody 124B11F6.
Figure 4E depicts humanization protocol of the heavy chain variable region of chimeric antibody 174C11C9.
Figure 4F depicts humanization protocol of the light chain variable region of chimeric antibody 174C11C9.
Figure 5A shows curve and EC50 values of humanized antibodies 21F6C6-hH1L1 and 21F6C6-hH2L2 reversing PBMC proliferation inhibition mediated by Siglec-15 protein.
Figure 5B shows curve and EC50 values of humanized antibodies 21F6C6-hH2L3 and 21F6C6-hH3L3 reversing inhibition of PBMC proliferation mediated by Siglec-15 protein.
Figure 5C shows curve and EC50 values of humanized antibodies 21F6C6-hH2L4 and 21F6C6-hH4L4 reversing inhibition of PBMC proliferation mediated by Siglec-15 protein.
Figure 5D shows curve and EC50 values of humanized antibodies 124B11F6-hH1L1, 124B11F6-hH2L2, and 124B11F6-hH3L3 reversing inhibition of PBMC proliferation mediated by Siglec-15 protein.
Figure 5E shows curve and EC50 values of humanized antibodies 174C11C9-hH1L1, 174C11C9-hH2L2, and 174C11C9-hH3L3 reversing inhibition of PBMC proliferation mediated by Siglec-15 protein.
Figure 6 shows humanized anti-Siglec-15 antibodies inhibited Siglec-15 protein mediated monocyte proliferation.
Figure 7 shows the tumor growth curves of mice in control and treatment groups in a MC38-hSiglec15 tumor model.
Figure 8 shows the body weight changes of mice over the treatment period in control and treatment groups in a MC38-hSiglec15 tumor model.

### Embodiments of the Invention

The present invention is illustrated further by way of examples. However, the scope of the invention is not limited to the examples. Experiments not specifically described in the following examples were carried out by conventional methods and under conventional conditions, or according to manufacturer's specification.

### Example 1. Preparation of Siglec-15 antigen and protein for detection

Gene sequences encoding the Siglec-15 antigen and protein for detection used in the present invention were obtained using the extracellular domain (F20-F263) of human Siglec-15 (UniProt Sialic acid-binding Ig-like lectin 15 Isoform 1) as template. The gene sequences were then recombined with an Fc fragment of antibody (e.g., human IgG1) or a His tag, expressed by HEK293 or Expi293 cells, and purified, to obtain the Siglec-15 antigen and the protein for detection used in the present invention. The antigen and the protein were purchased from ACROBiosystems (Beijing, China) under catalog # SG5-H5253 (Fc tag) and SG5-H52H3 (His tag). Human Siglec-15 extracellular domain has a sequence shown in SEQ ID NO:75, in which amino acids (aa) 1 to 19 represent signal peptide, aa 40 to 158 represent "V-set" domain, and aa 168 to 251 represent "C2-set" domain. The recombinant protein produced by linking human Siglec-15 full-length extracellular domain with human IgG1 Fc fragment, abbreviated as hS15-hFc, has a sequence shown in SEQ ID NO: 76, in which aa 1 to 244 represent human Siglec-15 full-length extracellular domain, and aa 253 to 483 represent human IgG1 Fc. The recombinant protein produced by linking Domain 1 (containing "V-set" domain) of Siglec-15 with human IgG1 Fc, abbreviated as hS15-D1-hFc, has a sequence shown in SEQ ID NO: 77, in which aa 1 to 4 represent signal peptide, aa 5 to 152 represent Domain 1 of Siglec-15 (in which aa 25 to 143 represent "V-set" domain of Siglec-15), aa 153 to 159 are linker sequence, and aa 160 to 390 represent human IgG1 Fc. The protein produced by linking human Siglec-15 full-length extracellular domain with His tag has a sequence shown in SEQ ID NO: 78, in which aa 1 to 244 represent human Siglec-15 full-length extracellular domain, and aa 253 to 262 represent His tag. Mouse and Cynomolgus Monkey Siglec-15 proteins useful for detection were commercially available and purchased from ACROBiosystems (Beijing, China) under catalog # SG5-M52H7and SG5-C52H6. The protein produced by linking Cynomolgus Monkey Siglec-15 full-length extracellular domain with His tag has a sequence shown in SEQ ID NO: 79, in which aa 1 to 244 represent monkey Siglec-15 full-length extracellular domain, and aa 253 to 262 represent His tag. The protein produced by linking mouse Siglec-15 full-length extracellular domain with His tag has a sequence shown in SEQ ID NO: 80, in which aa 1 to 239 represent mouse Siglec-15 full-length extracellular domain, and aa 248 to 257 represent His tag.

### Example 2. Preparation of hybridomas producing anti-human Siglec-15 monoclonal antibodies

Fifteen mice (5 C57bl/6, 5 balb/c, and 5 SJL) were immunized with hS15-hFc emulsified with adjuvant and, 2 weeks later, boosted with hS15-hFc or hS15-D1-hFc. Serum was taken for ELISA assay to assess antiserum titers. Mice with higher serum titers were selected for splenocytes fusion. Spleen cells were collected 3 days after the last booster immunization and fused by electrofusion for a total of 5 rounds. Each hybridoma cell obtained after fusion was plated on fifty 96-well plates. Positive clones were screened out based on binding activity experiments performed on hybridoma supernatant, and subject to subcloning. Positive clones that were then screened out based on experiments of binding activity and in vitro immune cell function were sequenced.

Results. Five positive hybridoma clones, *i.e.,* 21F6C6, 75E1F2, 124B11F6, 174C11C9, and 255C12B10, were screened out based on binding activity and in vitro immune cell function experiments, and sequences of the heavy chain variable regions and light chain variable regions of the five hybridoma clones were shown below.

### 21F6C6:

Sequence of Heavy Chain Variable Region (SEQ ID NO: 41):

EVMLVESGGGLVKPGGSLKLSCAASGFTFSSYAMSWVRQSPEKRLE
WVATISSGGSLIYYPDSVTGRFTISRDNAKSTLYLQMSSLRSEDTAMYYCVRH
GYYYGDSTLYYAMDYWGPGTSVTVSS

HCDR1, HCDR2, and HCDR3 are underlined, and amino acid sequences are shown in SEQ ID NOs: 1, 2, and 5. Sequences shown in SEQ ID NOs: 3 and 4 were obtained by one or two amino acid substitutions on the sequence of SEQ ID NO: 2.

Sequence of Light Chain Variable Region (SEQ ID NO: 42)

DIVLTQSPASLAVSLGQRATISCKASQSVDYDGDSYMNWYQQKPGQ
PPKLLIYAASNLESGIPARFSGSGSGTDFTLNIHPVEEEDAATYYCQQSNEAPL
TFGAGTKLELK

LCDR1, LCDR2, and LCDR3 are underlined, and amino acid sequences are shown in SEQ ID NOs: 6, 8, and 10. Sequences shown in SEQ ID NOs: 7 and 9 were obtained by one amino acid substitution on the sequences of SEQ ID NO: 6 and 8, respectively.

### 75E1F2:

Sequence of Heavy Chain Variable Region (SEQ ID NO: 43):

EVQLVESGGGSVKPGGSLKLSCAASGFTFSDYGMHWVRQAPEKGLE
WITYISSGSSIIDYSDTVKGRFTISRDNAKDTLFLQMTSLRSEDTAIYYCVRRED
HYAMDYWGQGTSVTVSS

HCDR1, HCDR2, and HCDR3 are underlined, and amino acid sequences are shown in SEQ ID NOs: 11, 12, and 13.

Sequence of Light Chain Variable Region (SEQ ID NO: 44)

DIVMTQSHKFLSTSVGDRVSITCKASQDVYTAVAWFQQKPGQSPKLL
FYSASYRFTGVPDRFNGSGSGTDFTLTITSMQAEDLAVYYCQQHYSTPWTFG
GGTKLEIK

LCDR1, LCDR2, and LCDR3 are underlined, and amino acid sequence are shown in SEQ ID NOs: 14, 15, and 16.

### 124B11F6:

Sequence of Heavy Chain Variable Region (SEQ ID NO: 45):

EVQLQQSGAELVRPGASVKLSCTASVFNIKDYYMHWVKQRTEQGLE
WIGRIDPEDDKTKYAPKFQGKATITADTSSNTAYLHLSTLTSEDTAVYYCVRG
WLGYIMDFWGQGTSVTVSS

HCDR1, HCDR2, and HCDR3 are underlined, and amino acid sequences are shown in SEQ ID NOs: 17, 18, and 20. Sequence shown in SEQ ID NO: 19 was obtained by two amino acid substitutions on the sequence of SEQ ID NO: 18.

Sequence of Light Chain Variable Region (SEQ ID NO: 46)

QLVLTQSSSASFSLGASAKLTCTLSSQHSTYTIDWYQQQPLKPPKYV
MELKEDGSHSTGDGIPDRFSGSSSGADRYLGISNIQPEDEAVYICGVGATIKEQ
FVYVFGGGTKVTVL

LCDR1, LCDR2, and LCDR3 are underlined, and amino acid sequences are shown in SEQ ID NOs: 21, 22, and 24. Sequence shown in SEQ ID NO: 23 was obtained by one amino acid substitution on the sequence of SEQ ID NO: 22.

### 174C11C9:

Sequence of Heavy Chain Variable Region (SEQ ID NO: 47):

EVQLQQSGPVLVKPGASVKMSCEASGYIFTDYYMNWVKQSPGKSLE
WIGVFNPYSGGISYNQKFKGKATLTVDTSSSTAYMELNSLTSEDSAVYYCARD
YYYLSSHGFYALDYWGQGTSVTVSS

HCDR1, HCDR2, and HCDR3 are underlined, and amino acid sequences are shown in SEQ ID NOs: 25, 26, and 29. Sequences shown in SEQ ID NOs: 27 and 28 were obtained by one or two amino acid substitutions on the sequence of SEQ ID NO: 26.

Sequence of Light Chain Variable Region (SEQ ID NO: 48)

DIVLTQSPASLAVSLGQRATISCRASESVDSYGNSFIHWYQQRPGRPP
KLLMYRASNLESGIPARFSGSGSRTDFTLTIDPVEADDVATYYCQQSNEDPLT
FGAGTKLELK

LCDR1, LCDR2, and LCDR3 are underlined, and amino acid sequences are shown in SEQ ID NOs: 30, 32, and 34. Sequences shown in SEQ ID NOs: 31 and 33 were obtained by one amino acid substitution on the sequences of SEQ ID NOs: 30 and 32, respectively.

### 255C12B10:

Sequence of Heavy Chain Variable Region (SEQ ID NO: 49):

EVQLQQSGPELVKPGASVKMSCKASGYTFTGYNMNWVKQSHGKSL
EWIGYINPNNGGTRYNQKFKGKATLTINKSSSTAYMELRSLTSEDSAVYYCA
RDYGIDYWGQGTTLTVSS

HCDR1, HCDR2, and HCDR3 are underlined, and amino acid sequences are shown in SEQ ID NOs: 35, 36, and 37.

Sequence of Light Chain Variable Region (SEQ ID NO: 50)

EIQMTQSPSSMSASLGDRITITCQATQDIVKNLNWYQQKPGKPPSFLI
YYATELAEGVPSRFSGSGSGSDYSLTISNLESEDFADYYCLQFYEFPYTFGGGT
KLEIK

LCDR1, LCDR2, and LCDR3 are underlined, and amino acid sequence are shown in SEQ ID NOs: 38, 39, and 40.

Note that the underlined sequences are CDR1, CDR2, and CDR3 of heavy or light chain variable region, and separated by the three CDR sequences are four framework regions of the heavy or light chain variable region, successively, FWR1, FWR2, FWR3, and FWR4.

**Table 1. CDR sequences of antibodies**

| Antibodies | Heavy Chain Variable Region No. | HCDR1 | SEQ ID NO: | HCDR2 | SEQ ID NO: | HCDR3 | SEQ ID NO: |
|---|---|---|---|---|---|---|---|
| 21F6C6 | 41 | SYAMS | 1 | TISSGGSLIY YPDSVTG | 2 | HGYYYGDST LYYAMDY | 5 |
| 75E1F2 | 43 | DYGMH | 11 | YISSGSSIID YSDTVKG | 12 | REDHYAMD Y | 13 |
| 124B11F6 | 45 | DYYMH | 17 | RIDPEDDKT KYAPKFQG | 18 | GWLGYIMDF | 20 |
| 174C11C9 | 47 | DYYMN | 25 | VFNPYSGGI SYNQKFKG | 26 | DYYYLSSHG FYALDY | 29 |
| 255C12B10 | 49 | GYNMN | 35 | YINPNNGGT RYNQKFKG | 36 | DYGIDY | 37 |
| Antibodies | Light Chain Variable Region No. | LCDR1 | | LCDR2 | | LCDR3 | |
| 21F6C6 | 42 | KASQSVDYD GDSYMN | 6 | AASNLES | 8 | QQSNEAPLT | 10 |
| 75E1F2 | 44 | KASQDVYTA VA | 14 | SASYRFT | 15 | QQHYSTPWT | 16 |
| 124B11F6 | 46 | TLSSQHSTYT ID | 21 | LKEDGSHST GD | 22 | GVGATIKEQ FVYV | 24 |
| 174C11C9 | 48 | RASESVDSY GNSFIH | 30 | RASNLES | 32 | QQSNEDPLT | 34 |
| 255C 12B 10 | 50 | QATQDIVKN LN | 38 | YATELAE | 39 | LQFYEFPYT | 40 |

### Example 3. Anti-human Siglec-15 antibodies bound to Siglec-15 protein from different species.

The variable regions from the antibodies produced by the hybridomas were combined with human IgG1 (N297A) constant regions to generate chimeric antibodies, named as 21F6C6, 75E1F2, 124B11F6, 174C11C9, and 255C12B10, respectively. Binding activity assays were performed on the chimeric antibodies using Siglec-15 proteins from different species, in which human, mouse and Cynomolgus Monkey Siglec-15 proteins were purchased from ACROBiosystems (Beijing, China). Antigen was coated at 0.5 µg/ml, and antibodies were diluted starting from 1 µg/ml and incubated with antigen. Horseradish peroxidase-labeled Anti-Human IgG antibody was used as the secondary antibody and OD₄₅₀ values were read after adding the substrate.

Results. All 5 anti-Siglec-15 chimeric antibodies bound to both human and Cynomolgus Monkey Siglec-15, and chimeric antibodies originated from hybridomas 124B11F6, 174C11C9, and 255C12B10 bound to mouse Siglec-15. Table 2 shows the EC₅₀ values of the anti-Siglec-15 chimeric antibodies binding to Siglec-15 antigens from different species.

**Table 2. EC₅₀ values of anti-Siglec-15 chimeric antibodies binding to Siglec-15 antigens from different species.**

| Antibodies | EC50 (ng/ml) | | |
|---|---|---|---|
| | Human Siglec-15 | Mouse Siglec-15 | Cynomolgus Monkey Siglec-15 |
| 21F6C6 chimeric antibody | 4.808 | N/A | 6.606 |
| 75E1F2 chimeric antibody | 18.57 | N/A | 12.17 |
| 124B11F6 chimeric antibody | 21.56 | 21.22 | 15.99 |
| 174C11C9 chimeric antibody | 5.218 | 9.855 | 7.484 |
| 255C12B10 chimeric antibody | 5.623 | 22.82 | 5.456 |

### Example 4. Binding of anti-human Siglec-15 antibodies to cells expressing human Siglec-15

HEK293T cell line overexpressing Siglec-15 (HEK293T-hu-S15) was purchased from KYinno Biotechnology Co., Ltd (Beijing, China) under Catalog # KC-1314. Purified anti-Siglec-15 chimeric antibodies were diluted at a gradient of 100 nM and incubated with HEK293T-hu-S15 cells at 4°C for 30 min. Flow cytometry was performed after addition of anti-human IgG (Fc specific)-FITC (cat. # F9512) and incubation for 30 min.

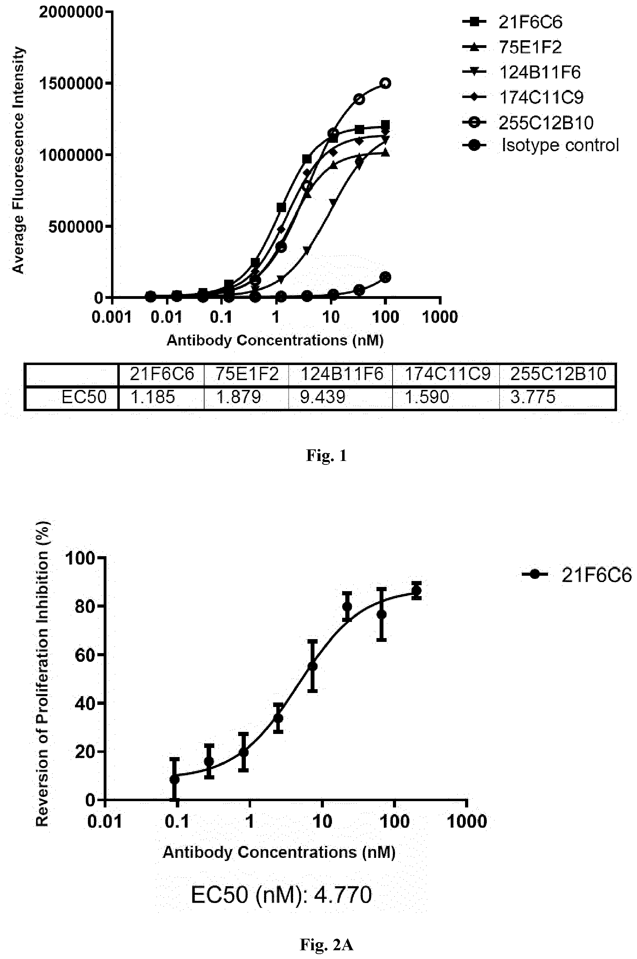

Results. All 5 anti-Siglec-15 chimeric antibodies bound to HEK293T-hu-S15 cell line. Figure 1 shows the binding curves and EC50 values of the anti-Siglec-15 chimeric antibodies to HEK293T-hu-S15 cell line.

### Example 5. Affinity Assessment on recombinant antibodies to Siglec-15 protein

Affinity analysis was performed using Biacore8K, and a CM5 chip immobilized with anti-human Fc antibody was used to capture the antibodies tested. The stock solutions of the antibodies to be tested were diluted to 5 µg/mL, and injected sequentially into the experimental channels at a flow rate of 10 µL/min at about 500 RU. The analyte was Human Siglec-15, His Tag (Cat.# SG5-H52H3), the diluted human Siglec-15 protein was injected sequentially into the experimental channels and the control channel at a flow rate of 30 µL/min and allowing for a binding time of 150 s and a dissociation time of 700 s. The KD value of each antibody was calculated using Biacore 8K analysis software.

Results. Table 3 shows the affinity analysis results of anti-Siglec-15 chimeric antibodies to Siglec-15 protein.

**Table 3. Affinity analysis results of anti-Siglec-15 chimeric antibodies to Siglec-15 protein.**

| Antibodies | ka (1/Ms) | kd (1/s) | KD (M) |
|---|---|---|---|
| 21F6C6 chimeric antibody | 3.95E+06 | 1.87E-04 | 4.73E-11 |
| 174C11C9 chimeric antibody | 1.21E+06 | 3.70E-04 | 3.05E-10 |
| 75E1F2 chimeric antibody | 8.41E+05 | 7.82E-04 | 9.30E-10 |
| 124B11F6 chimeric antibody | 1.02E+05 | 1.46E-07 | 1.43E-12 |
| 255C12B10 chimeric antibody | 2.96E+05 | 1.58E-04 | 5.36E-10 |

### Example 6. Epitope Mapping of the Chimeric Antibodies

Epitope binning assays were performed using the Octet RED96e (ForteBio) System for the antibodies tested. Human Siglec-15, His Tag (Cat. No. SG5-H52H3) (5 µg/mL) was captured by the HIS 1K biosensor and bound to primary antibody (100 nM) for 180 s. Then, the Human Siglec-15, His Tag (antigen)-primary antibody was bound to secondary antibody (100 nM) on the biosensors for 90 s. Data analysis was performed using Octet RED96e (ForteBio) analysis software.

Results. Table 4 shows the cross-competition scores among the anti-Siglec-15 chimeric antibodies. 60%-100% represent completely non-competitive, indicating different or partially different epitopes; 20%-60% represent partially competitive, indicating partially different epitopes; and <20% represent completely competitive, indicating same epitope.

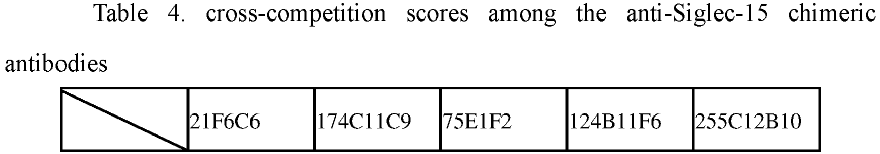

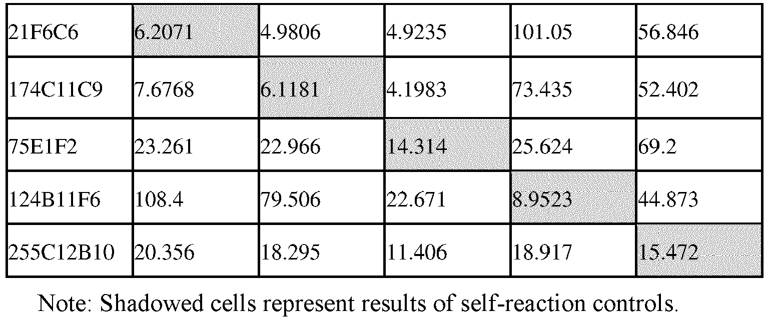

### Example 7. Anti-Siglec-15 chimeric antibodies could reverse the inhibition of PBMC proliferation mediated by Siglec-15 protein.

Cryopreserved peripheral blood mononuclear cells (PBMCs) were resuscitated, and gradient diluted anti-Siglec-15 chimeric antibodies were incubated with hS15-hFc protein (0.5 µg/ml) at 37°C for 30 minutes before adding 1×10⁵ PBMC cells and soluble OKT3 (0.5 ng/ml, eBioscience). Three days after incubation, celltiterglo (Promega) was added, and fluorescence values were read to evaluate cell proliferation.

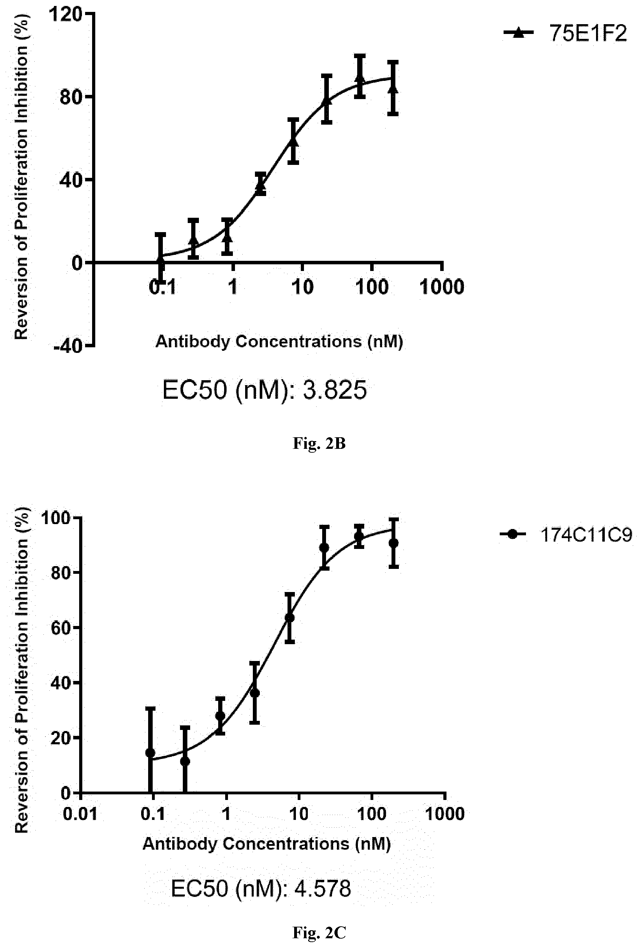

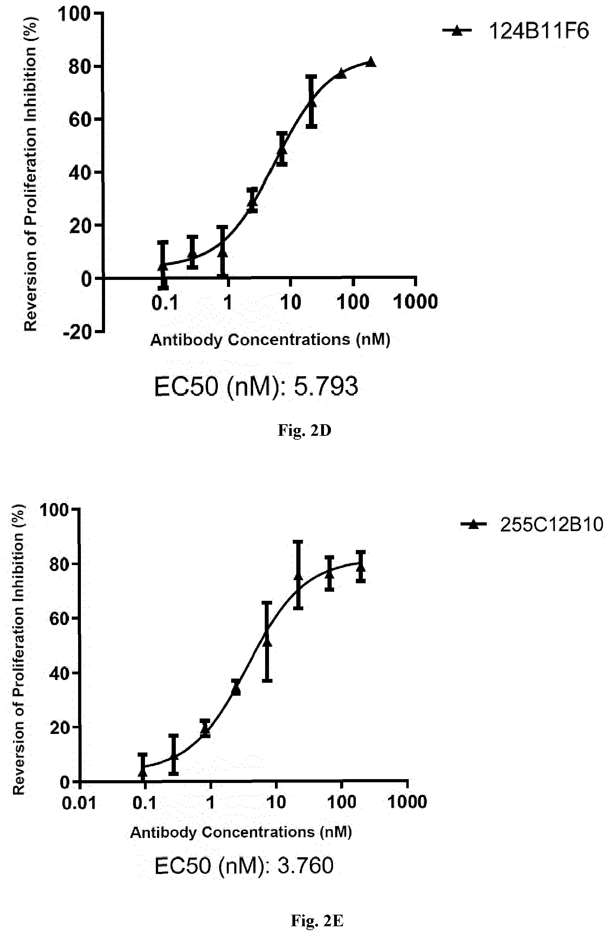

Results. All 5 anti-Siglec-15 chimeric antibodies could reverse the inhibition of PBMC proliferation mediated by hS15-hFc. Figures 2A-E are curves plotting the reversion percent of inhibition by antibodies 21F6C6, 75E1F2, 174C11C9, 124B11F6, and 255C12B10, and EC50 values.

### Example 8. Anti-Siglec-15 chimeric antibodies could inhibit monocyte survival mediated by Siglec-15 protein.

Cryopreserved CD14+ monocytes isolated from human PBMCs were resuscitated, and different concentrations of anti-Siglec-15 chimeric antibodies were incubated with hS15-hFc protein at 37°C for 30 min before adding 1×10⁵ CD14+ monocytes. After 6 days of culture, XTT (Roche, catalog number 11465015001) was added to the plate, and after 2 hours of incubation, OD450-OD690 was read to measure cell viability.

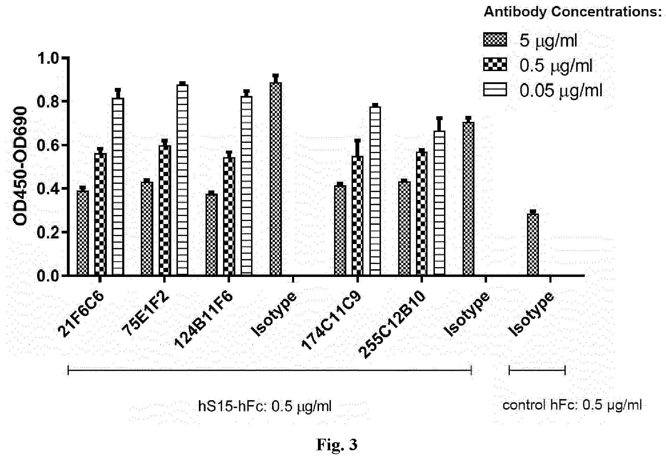

Results. Figure 3 shows hS15-hFc could induce CD14+ monocyte survival and proliferation, and anti-Siglec-15 chimeric antibodies could inhibit monocyte proliferation induced by Siglec-15 protein in a concentration dependent manner.

### Example 9. Humanization of anti-Siglec-15 antibodies

Chimeric antibodies 21F6C6, 124B11F6, and 174C11C9 were humanized with four heavy chains and four light chains. See Table 5 below.

**Table 5. Humanization of Chimeric Antibodies**

| Humanized Heavy Chain Variable Regions | SEQ ID NO: | | | |
|---|---|---|---|---|
| | Heavy Chain Variable Regions | HCDR1 | HCDR2 | HCDR3 |
| 21F6C6-mVH (mouse antibody, parent) | 41 | 1 | 2 | 5 |
| 21F6C6-hVH1 | 51 | 1 | 2 | 5 |
| 21F6C6-hVH2 | 53 | 1 | 3 | 5 |
| 21F6C6-hVH3 | 55 | 1 | 4 | 5 |
| 21F6C6-hVH4 | 57 | 1 | 4 | 5 |
| 124B11F6-mVH (mouse antibody, parent) | 45 | 17 | 18 | 20 |
| 124B11F6-hVH1 | 59 | 17 | 18 | 20 |
| 124B 11F6-hVH2 | 61 | 17 | 18 | 20 |
| 124B 11F6-hVH3 | 63 | 17 | 18 | 20 |
| 124B11F6-hVH4 | 65 | 17 | 19 | 20 |
| 174C11C9-mVH (mouse antibody, parent) | 47 | 25 | 26 | 29 |
| 174C11C9-hVH1 | 67 | 25 | 26 | 29 |
| 174C11C9-hVH2 | 69 | 25 | 26 | 29 |
| 174C11C9-h1VH3 | 71 | 25 | 27 | 29 |
| 174C11C9-hVH4 | 73 | 25 | 28 | 29 |

| Humanized Light Chain Variable Regions | SEQ ID NO: | | | |
|---|---|---|---|---|
| | Light Chain Variable Regions | LCDR1 | LCDR2 | LCDR3 |
| 21F6C6-mVL (mouse antibody, parent) | 42 | 6 | 8 | 10 |
| 21F6C6-hVL1 | 52 | 7 | 8 | 10 |
| 21F6C6-hVL2 | 54 | 7 | 8 | 10 |
| 21F6C6-hVL3 | 56 | 7 | 8 | 10 |
| 21F6C6-hVL4 | 58 | 7 | 9 | 10 |
| 124B11F6-mVL (mouse antibody, parent) | 46 | 21 | 22 | 24 |
| 124B11F6-hVL1 | 60 | 21 | 22 | 24 |
| 124B 11F6-h VL2 | 62 | 21 | 22 | 24 |
| 124B 11F6-hVL3 | 64 | 21 | 23 | 24 |
| 124B 11F6-hVL4 | 66 | 21 | 23 | 24 |
| 174C11C9-mVL (mouse antibody, parent) | 48 | 30 | 32 | 34 |
| 174C11C9-hVL1 | 68 | 30 | 32 | 34 |
| 174C11C9-hVL2 | 70 | 30 | 32 | 34 |
| 174C11C9-hVL3 | 72 | 30 | 32 | 34 |
| 174C11C9-hVL4 | 74 | 31 | 33 | 34 |

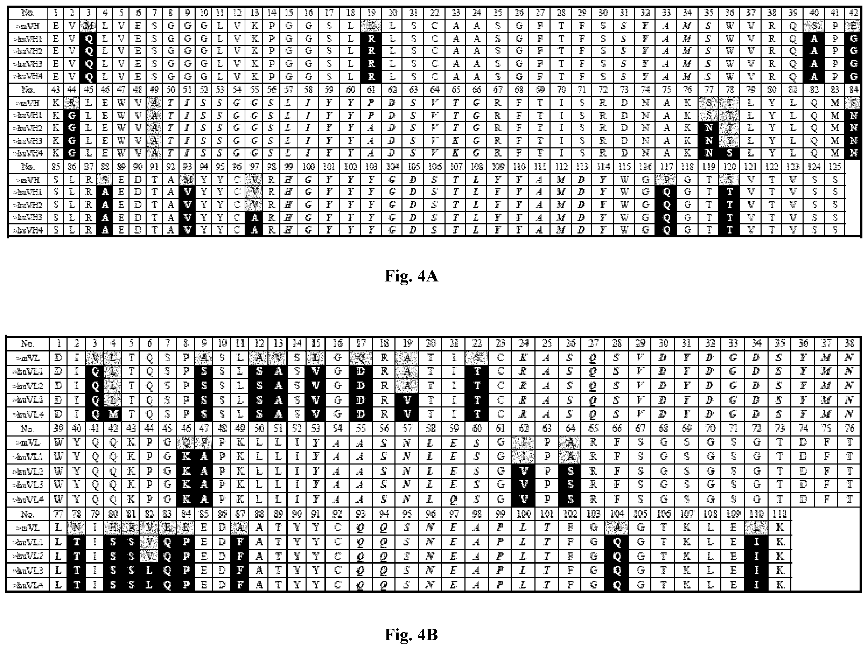

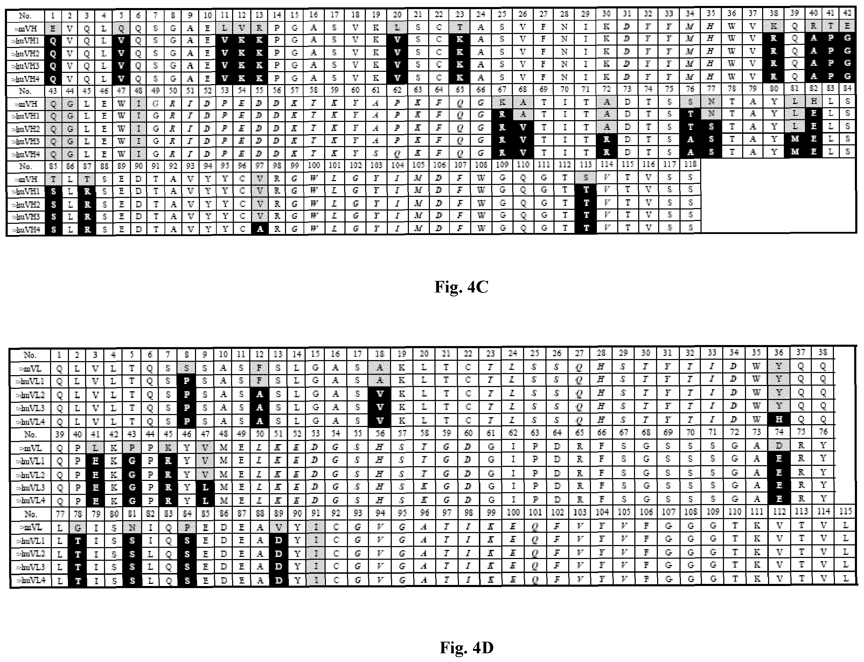

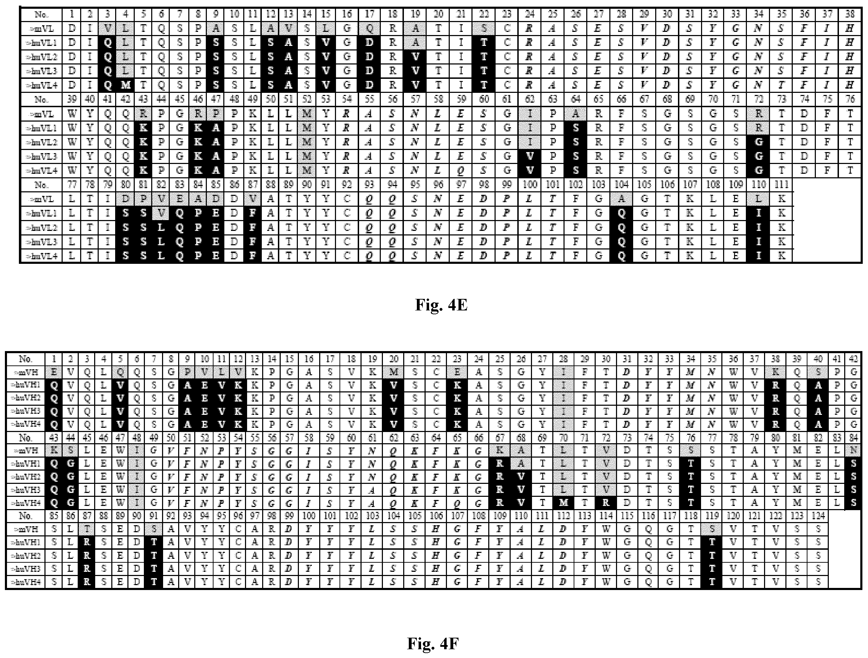

Results. Humanization of 21F6C6 is shown in Figures 4A and 4B, humanization of 124B11F6 is shown in Figures 4C and 4D, and humanization of 174C11C9 is shown in Figures 4E and 4F. Grey zones indicate mouse sequences, Bold and Italic zones indicate CDR regions, Black zones indicate humanized sequences, and the rest zones are original human sequences. In figures 4A to 4F, the abbreviation "hu" for humanized antibodies has the same meaning as the letter "h" in Table 5, and they can be used interchangeably herein and throughout the disclosure of the invention.

### Example 10. Affinity Assessment of Humanized Antibodies to Siglec-15 protein

The humanized sequences of 21F6C6, 124B11F6, and 174C11C9 were expressed in different combinations (Table 6). Humanized antibody 21F6C6-hH2L4V was obtained via site-specific mutagenesis in LCDR1 of 21F6C6-hH2L4, with post-translational isomerization removed. The light chain variable region is shown in SEQ ID NO: 82 (21F6C6-hH2L4V), and the LCDR1 sequence is shown in SEQ ID NO: 81 (21F6C6-hH2L4V).

Affinity analysis was performed using Biacore. A CM5 chip immobilized with anti-human Fc antibody was used to capture the antibodies tested, and the analyte was Siglec-15 protein. (See Example 6 for methods.)

Results. Table 6 shows the affinity analysis results of the humanized antibodies to Siglec-15 protein.

**Table 6. Affinity analysis results of the humanized antibodies to Siglec-15 protein**

| Humanized Antibodies | Heavy Chain Variable Region SEQ ID NO: | Light Chain Variable Region SEQ ID NO: | Heavy Chain Constant Region Isotype | Light Chain Constant Region Isotype | ka (1/Ms) | kd (1/s) | KD (M) |
|---|---|---|---|---|---|---|---|
| 21F6C6-mHL | 41 | 42 | hIgG 1 | Kappa | 5.49E+06 | 3.75E-04 | 6.84E-11 |
| 21F6C6-hH1L1 | 51 | 52 | hIgG 1 | Kappa | 4.23E+06 | 3.41E-04 | 8.06E-11 |
| 21F6C6-hH2L2 | 53 | 54 | hIgG 1 | Kappa | 4.59E+06 | 3.14E-04 | 6.83E-11 |
| 21F6C6-hH2L3 | 53 | 56 | hIgG 1 | Kappa | 4.87E+06 | 3.53E-04 | 7.24E-11 |
| 21F6C6-hH2L4 | 53 | 58 | hIgG 1 | Kappa | 4.84E+06 | 4.14E-04 | 8.56E-11 |
| 21F6C6-hH2L4V | 53 | 82 | hIgG 1 | Kappa | 1.61E+06 | 1.84E-04 | 1.14E-10 |
| 124B11F6-mHL | 45 | 46 | hIgG 1 | Lambda | 1.37E+06 | 1.14E-04 | 8.34E-11 |
| 124B11F6-hH1L1 | 59 | 60 | hIgG 1 | Lambda | 1.17E+06 | 1.27E-04 | 1.09E-10 |
| 124B 11F6-hH2L2 | 61 | 62 | hIgG 1 | Lambda | 1.11E+06 | 2.24E-04 | 2.02E-10 |
| 174C11C9-mHL | 47 | 48 | hIgG 1 | Kappa | 1.74E+06 | 3.05E-04 | 1.75E-10 |
| 174C11C9-hH2L2 | 69 | 70 | hIgG 1 | Kappa | 1.37E+06 | 4.47E-04 | 3.25E-10 |
| 174C11C9-hH3L3 | 71 | 72 | hIgG 1 | Kappa | 7.85E+05 | 4.82E-04 | 6.14E-10 |

21F6C6-mHL represents chimeric antibody 21F6C6 consisting of mouse variable regions and hIgG1 (similarly for 174C11C9-mHL and 124B11F6-mHL). 21F6C6-hH1L1 represents a humanized antibody recombined by huVH1 in figure 4A, huVL1 in figure 4B, hIgG1 heavy chain constant region, and Kappa light chain constant region. 124B11F6-hH1L1 represents a humanized antibody recombined by huVH1 in figure 4C, huVL1 in figure 4D, hIgG1 heavy chain constant region, and Lambda-C2 light chain constant region. 174C11C9-hH2L2 represents a humanized antibody recombined by huVH2 in figure 4E, huVL2 in figure 4F, hIgG1 heavy chain constant region, and Kappa light chain constant region. Other antibodies are similarly designated.

### Example 11. Humanized Antibodies could reverse the inhibition of Siglec-15-mediated PBMC proliferation.

Cryopreserved peripheral blood mononuclear cells (PBMCs) were resuscitated, and gradient diluted anti-Siglec-15 humanized antibodies were incubated with hS15-hFc protein (1 µg/ml) at 37°C for 30 minutes before adding 1×10⁵ PBMC cells and soluble OKT3 (5 ng/ml, eBioscience). Three days after incubation, celltiterglo (Promega) was added, and fluorescence values were read to evaluate cell proliferation.

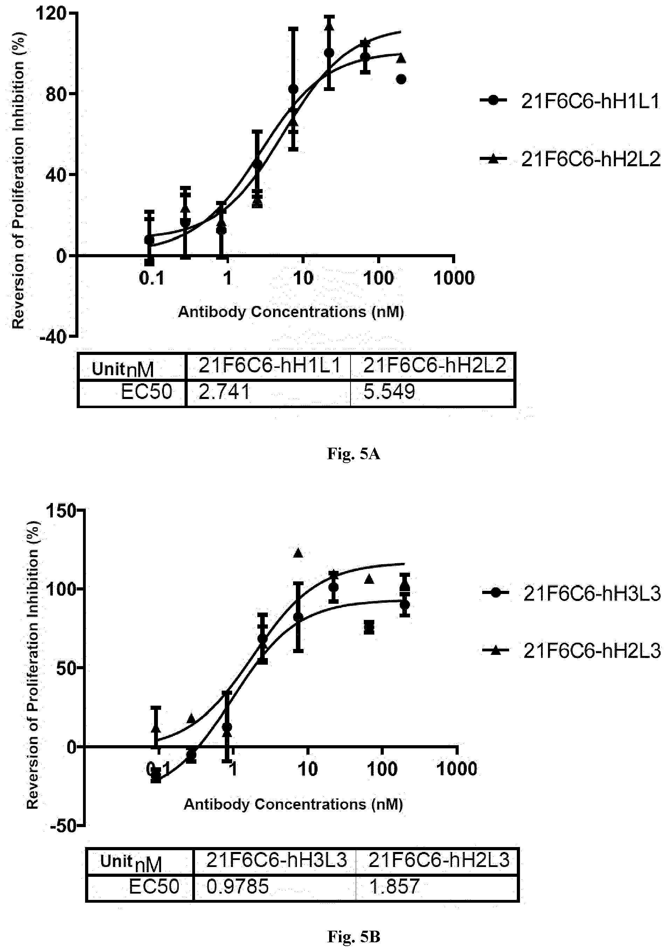

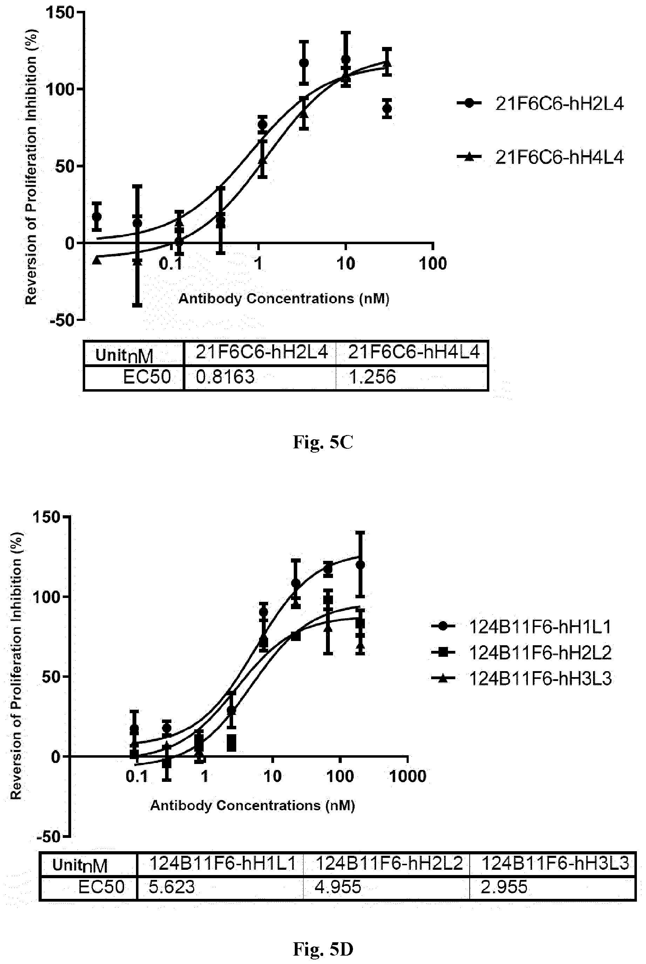

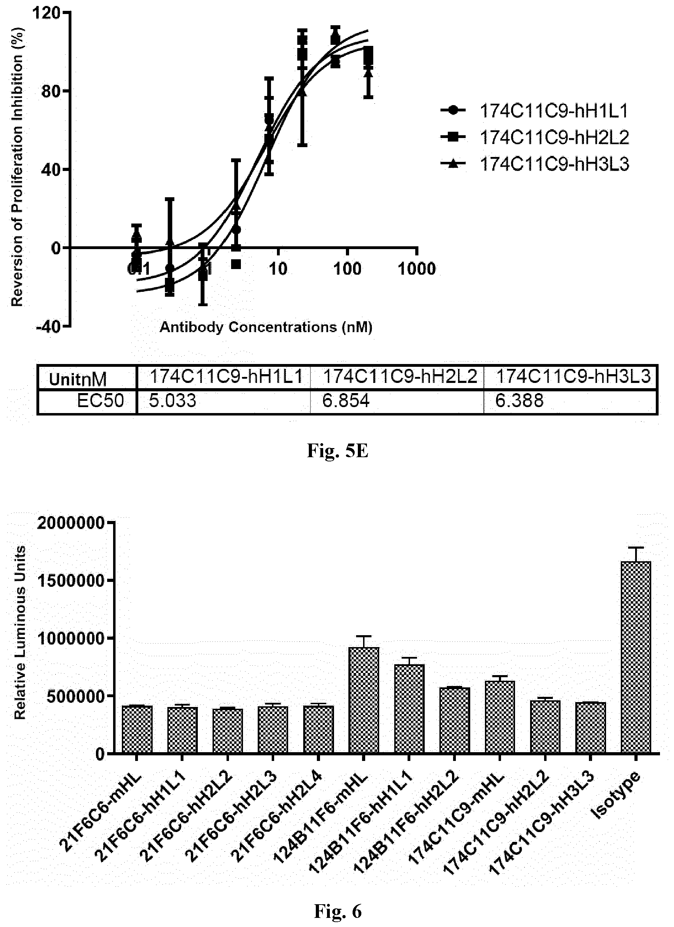

Results. Anti-Siglec-15 humanized antibodies could reverse the inhibition of PBMC proliferation mediated by hS15-hFc. Figures 5A-E are curves plotting the reversion percent of inhibition by antibodies, and EC50 values.

### Example 12. Anti-Siglec-15 humanized antibodies could inhibit Siglec-15-mediated monocyte survival.

Cryopreserved CD14+ monocytes isolated from human PBMCs were resuscitated, and anti-Siglec-15 humanized antibodies (10 µg/ml) were incubated with hS15-hFc protein (2 µg/ml) at 37°C for 30 min before adding 1×10⁵ CD14+ monocytes. After 5 days of culture, celltiterglo (Promega) was added, and fluorescence values were read to evaluate cell viability.

Results. Figure 6 shows anti-Siglec-15 humanized antibodies could inhibit Siglec-15 protein-mediated monocyte proliferation.

### Example 13. Anti-tumor efficacy evaluation of anti-Siglec-15 humanized antibodies in a HuCELL tumor model

Fifty SIGLEC15 humanized C57BL/6J mice (C57BL/6J-SIGLEC 15HuGEMM), female, 6~8 weeks (mouse age at the time of tumor inoculation), weighted 18-24g (body weight at the time of grouping) were purchased from Shanghai South Model Animal Center (SMOC), with animal certificate number of 20190002004257,. Thirty-two tumor-bearing mice were randomly grouped according to body weight and tumor size, respectively. Mice were housed in a SPF-grade environment.

Murine MC38-hSiglec15 cells (Clone #6, cell number ECL-00260) were cultured in DMEM medium containing 10% fetal bovine serum. MC38-hSiglec15 cells from the exponential growth phase were collected for seeding. The cells were resuspended in PBS, and each mouse was subcutaneously inoculated at 5×10⁶ cells/100 µL at the right shoulder blade on the back. C57BL/6J-SIGLEC15HuGEM mice were inoculated with MC38-hSiglec15 tumor cells subcutaneously to establish an allograft tumor model of colon cancer cells. The control group was hIgG 1 isotype, and the treatment groups were 21F6C6-hH2L4V (10mg/kg), 174C11C9-hH2L2 (10mg/kg), with 8 mice in each group; The control group and the treatment groups were all administered via intraperitoneal injection, and the efficacy was evaluated based on tumor growth inhibition (TGI).

### 21F6C6-hH2L4V:

Sequence of Heavy Chain Variable Region (SEQ ID NO: 53)

EVQLVESGGGLVKPGGSLRLSCAASGFTFSSYAMSWVRQAPGKGLE
WVATISSGGSLIYYADSVTGRFTISRDNAKNTLYLQMNSLRAEDTAVYYCVR
HGYYYGDSTLYYAMDYWGQGTTVTVSS

HCDR1, HCDR2, and HCDR3 are underlined, and amino acid sequences are shown in SEQ ID NOs: 1, 3, and 5.

Sequence of Light Chain Variable Region (SEQ ID NO: 82)

DIQMTQSPSSLSASVGDRVTITCRASQSVDYEGDSYMNWYQQKPGK
APKLLIYAASNLQSGVPSRFSGSGSGTDFTLTISSLQPEDFATYYCQQSNEAPL
TFGQGTKLEIK

LCDR1, LCDR2, and LCDR3 are underlined, and amino acid sequences are shown in SEQ ID NOs: 81, 9, and 10.

### 174C11C9-hH2L2:

Sequence of Heavy Chain Variable Region (SEQ ID NO: 69)

QVQLVQSGAEVKKPGASVKVSCKASGYIFTDYYMNWVRQAPGQGL
EWIGVFNPYSGGISYNQKFKGRVTLTVDTSTSTAYMELSSLRSEDTAVYYCA

RDYYYLSSHGFYALDYWGQGTTVTVSS

HCDR1, HCDR2, and HCDR3 are underlined, and amino acid sequences are shown in SEQ ID NOs: 25, 26, and 29.

Sequence of Light Chain Variable Region (SEQ ID NO: 70)

DIQLTQSPSSLSASVGDRVTITCRASESVDSYGNSFIHWYQQKPGKAP
KLLMYRASNLESGIPSRFSGSGSGTDFTLTISSLQPEDFATYYCQQSNEDPLTF
GQGTKLEIK

LCDR1, LCDR2, and LCDR3 are underlined, and amino acid sequences are shown in SEQ ID NOs: 30, 32, and 34.

Tumor volume was calculated according to formula: tumor volume (mm³) = 1/2× (a×b2), where *a* represents long diameter and *b* represents short diameter. Data were collected using the StudyDirectorTM (version 3.1.399.19, StudylogSystem, Inc., S. San Francisco, CA, USA) software, including tumor long and short diameters, and animal weights. The tumor growth inhibition, TGI (%), was calculated as follows: TGI %=100× (1-ΔT/ΔC). (ΔT and ΔC were the mean tumor volume (MTVₜ) at a specific time point in the treatment and control groups, respectively, minus the mean tumor volume (MTV₁) when grouped).

All statistical analysis and graphical plotting were performed in the R programing language environment (version 3.3.1). Unless otherwise specified, all tests were two-tailed tests and considered statistically significant when the P value is less than 0.05.

### Experimental Results

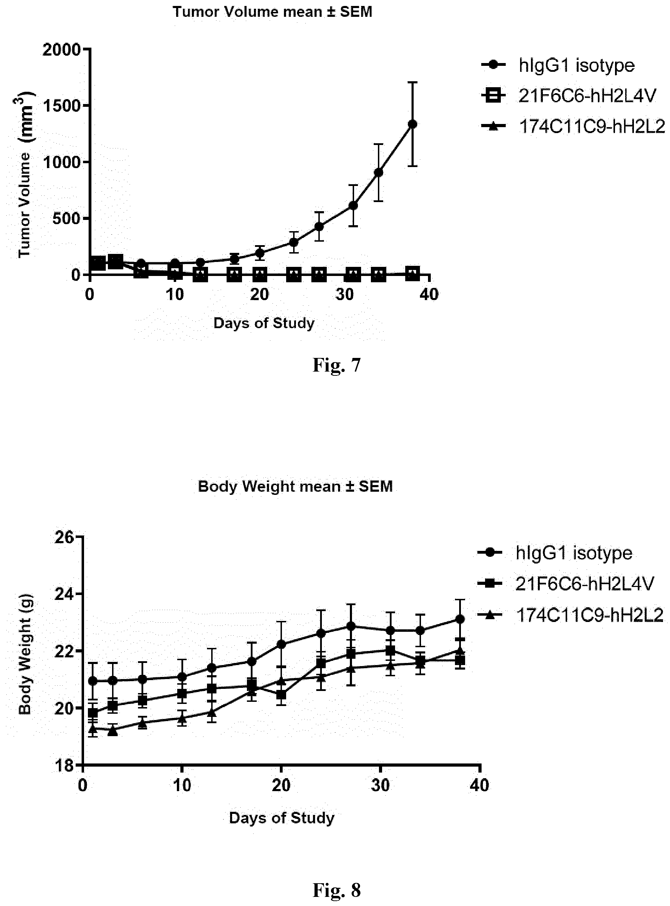

The average tumor volumes of mice on day 38 after treatment were analyzed. The average volume of the tumor at the right shoulder blade on the back in the hIgG1 isotype (10 mg/kg) control group was 1134 mm³ on day 38 after treatment. The tumors in the **21F6C6-hH2L4V** (10 mg/kg) treatment group began to disappear on day 6 after treatment, and all mice were cured on day 13 after treatment; the tumor volumes of 7 mice remained at 0 mm³, with one mouse tumor recurred. The overall tumor growth inhibition TGI was 107%, which was statistically significant compared with the control group in anti-tumor effect (P<0.005). The tumors began to disappear on day 6 after treatment in the **174C11C9-hH2L2** (10mg/kg) treatment group, and all mice were cured on day 13 after treatment; the tumor volume of all mice was 0 mm³ until day 38, and the tumor growth inhibition TGI was 108% which was statistically significant compared with the control group in anti-tumor effect (P<0.005). The tumor growth of the control group and the treatment groups is shown in Figure 7. The body weight change curves of the control group and the treatment groups plotted over treatment time are shown in Figure 8. The results of this study showed that, compared with the negative control MgG1 isotype (10mg/kg), **21F6C6-hH2L4V** (10mg/kg) alone, and **174C11C9-hH2L2** (10mg/kg) alone, exhibited significant anti-tumor effect (P<0.005) on MC38-hSiglec15 tumor model. The mice were generally cured, and there was basically no tumor recurrence after tumor disappearance within prolonged observation period.

## Claims

1. An isolated antibody binding to Siglec-15 or an antigen binding fragment thereof, comprising one or more CDR sequences selected from a group consisting of SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 81; preferably, the isolated antibody binding to Siglec-15 or an antigen binding fragment thereof comprises one or more heavy chain CDR sequences selected from a group consisting of SEQ ID NOs: 1, 2, 3, 4, 5, 11, 12, 13, 17, 18, 19, 20, 25, 26, 27, 28, 29, 35, 36, and 37, and/or, one or more light chain CDR sequences selected from a group consisting of SEQ ID NOs: 6, 7, 8, 9, 10, 14, 15, 16, 21, 22, 23, 24, 30, 31, 32, 33, 34, 38, 39, 40, and 81.

2. The isolated antibody binding to Siglec-15 or an antigen binding fragment thereof, according to claim 1, wherein the isolated antibody binding to Siglec-15 or an antigen binding fragment thereof comprises a heavy chain variable region comprising HCDR1, HCDR2 and HCDR3 sequences, and/or a light chain variable region comprising LCDR1, LCDR2 and LCDR3 sequences, wherein:
(a) the HCDR1 sequence of the heavy chain variable region comprises an amino acid sequence selected from a group consisting of SEQ ID NOs: 1, 11, 17, 25, 35, and a conservatively modified variant each thereof; the HCDR2 sequence of the heavy chain variable region comprises an amino acid sequence selected from a group consisting of SEQ ID NOs: 2, 3, 4, 12, 18, 19, 26, 27, 28, 36, and a conservatively modified variant each thereof; and the HCDR3 sequence of the heavy chain variable region comprises an amino acid sequence selected from a group consisting of SEQ ID NOs: 5, 13, 20, 29, 37, and a conservatively modified variant each thereof;
(b) the LCDR1 sequence of the light chain variable region comprises an amino acid sequence selected from a group consisting of SEQ ID NOs: 6, 7, 14, 21, 30, 31, 38, 81, and a conservatively modified variant each thereof; the LCDR2 sequence of the light chain variable region comprises an amino acid sequence selected from a group consisting of SEQ ID NOs: 8, 9, 15, 22, 23, 32, 33, 39, and a conservatively modified variant each thereof; and the LCDR3 sequence of the light chain variable region comprises an amino acid sequence selected from a group consisting of SEQ ID NOs: 10, 16, 24, 34, 40, and a conservatively modified variant each thereof; or
(a) the heavy chain variable region comprises a CDR sequence selected from a group consisting of SEQ ID NOs: 1, 2, 3, 4 and 5, and the light chain variable region comprises a CDR sequence selected from a group consisting of SEQ ID NOs: 6, 7, 8, 9, 10, and 81; or
(b) the heavy chain variable region comprises a CDR sequence selected from a group consisting of SEQ ID NOs: 11, 12, and 13, and the light chain variable region comprises a CDR sequence selected from a group consisting of SEQ ID NOs: 14, 15, and 16; or
(c) the heavy chain variable region comprises a CDR sequence selected from a group consisting of SEQ ID NOs: 17, 18, 19, and 20, and the light chain variable region comprises a CDR sequence selected from a group consisting of SEQ ID NOs: 21, 22, 23, and 24; or
(d) the heavy chain variable region comprises a CDR sequence selected from a group consisting of SEQ ID NOs: 25, 26, 27, 28, and 29, and the light chain variable region comprises a CDR sequence selected from a group consisting of SEQ ID NOs: 30, 31, 32, 33, and 34; or
(e) the heavy chain variable region comprises a CDR sequence selected from a group consisting of SEQ ID NOs: 35, 36, and 37, and the light chain variable region comprises a CDR sequence selected from a group consisting of SEQ ID NOs: 38, 39, and 40.

3. The isolated antibody binding to Siglec-15 or an antigen binding fragment thereof, according to claim 1, wherein the isolated antibody binding to Siglec-15 or an antigen binding fragment thereof comprises a heavy chain variable region comprising HCDR1, HCDR2 and HCDR3, and/or a light chain variable region comprising LCDR1, LCDR2 and LCDR3, wherein
(a) the HCDR1 sequence is shown in SEQ ID NO: 1, the HCDR2 sequence is selected from a group consisting of SEQ ID NOs: 2, 3, and 4, the HCDR3 sequence is shown in SEQ ID NO: 5, the LCDR1 sequence is selected from a group consisting of SEQ ID NOs: 6, 7, and 81, the LCDR2 sequence is selected from a group consisting of SEQ ID NOs: 8 and 9, and the LCDR3 sequence is shown in SEQ ID NO: 10; or
(b) the HCDR1 sequence is shown in SEQ ID NO: 11, the HCDR2 sequence is shown in SEQ ID NO: 12, the HCDR3 sequence is shown in SEQ ID NO: 13, the LCDR1 sequence is shown in SEQ ID NO: 14, the LCDR2 sequence is shown in SEQ ID NO: 15, and the LCDR3 sequence is shown in SEQ ID NO: 16; or
(c) the HCDR1 sequence is shown in SEQ ID NO: 17, the HCDR2 sequence is selected from a group consisting of SEQ ID NOs: 18 and 19, the HCDR3 sequence is shown in SEQ ID NO: 20, the LCDR1 sequence is shown in SEQ ID NO: 21, the LCDR2 sequence is selected from a group consisting of SEQ ID NOs: 22 and 23, and the LCDR3 sequence is shown in SEQ ID NO: 24; or
(d) the HCDR1 sequence is shown in SEQ ID NO: 25, the HCDR2 sequence is selected from a group consisting of SEQ ID NOs: 26, 27, and 28, the HCDR3 sequence is shown in SEQ ID NO: 29, the LCDR1 sequence is selected from a group consisting of SEQ ID NOs: 30 and 31, the LCDR2 sequence is selected from a group consisting of SEQ ID NOs: 32 and 33, and the LCDR3 sequence is shown in SEQ ID NO: 34; or
(e) the HCDR1 sequence is shown in SEQ ID NO: 35, the HCDR2 sequence is shown in SEQ ID NO: 36, the HCDR3 sequence is shown in SEQ ID NO: 37, the LCDR1 sequence is shown in SEQ ID NO: 38, the LCDR2 sequence is shown in SEQ ID NO: 39, and the LCDR3 sequence is shown in SEQ ID NO: 40.

4. The isolated antibody binding to Siglec-15 or an antigen binding fragment thereof, according to any one of claims 1 to 3, wherein the isolated antibody binding to Siglec-15 or an antigen binding fragment thereof may be a murine antibody, a chimeric antibody, a humanized antibody, a human antibody, or a fragment each thereof, and preferably, a humanized antibody or a fragment thereof.

5. The isolated antibody binding to Siglec-15 or an antigen binding fragment thereof, according to claim 1, wherein the isolated antibody binding to Siglec-15 or an antigen binding fragment thereof comprises
(a) a heavy chain variable region as shown in SEQ ID NO: 41 or having an amino acid sequence having at least 90%, 95%, 96%, 97%, 98%, or 99% homology to SEQ ID NO: 41; and/or a light chain variable region as shown in SEQ ID NO: 42 or having an amino acid sequence having at least 90%, 95%, 96%, 97%, 98%, or 99% homology to SEQ ID NO: 42; or
(b) a heavy chain variable region as shown in SEQ ID NO: 43 or having an amino acid sequence having at least 90%, 95%, 96%, 97%, 98%, or 99% homology to SEQ ID NO: 43; and/or a light chain variable region as shown in SEQ ID NO: 44 or having an amino acid sequence having at least 90%, 95%, 96%, 97%, 98%, or 99% homology to SEQ ID NO: 44; or
(c) a heavy chain variable region as shown in SEQ ID NO: 45 or having an amino acid sequence having at least 90%, 95%, 96%, 97%, 98%, or 99% homology to SEQ ID NO: 45; and/or a light chain variable region as shown in SEQ ID NO: 46 or having an amino acid sequence having at least 90%, 95%, 96%, 97%, 98%, or 99% homology to SEQ ID NO: 46; or
(d) a heavy chain variable region as shown in SEQ ID NO: 47 or having an amino acid sequence having at least 90%, 95%, 96%, 97%, 98%, or 99% homology to SEQ ID NO: 47; and/or a light chain variable region as shown in SEQ ID NO: 48 or having an amino acid sequence having at least 90%, 95%, 96%, 97%, 98%, or 99% homology to SEQ ID NO: 48; or
(e) a heavy chain variable region as shown in SEQ ID NO: 49 or having an amino acid sequence having at least 90%, 95%, 96%, 97%, 98%, or 99% homology to SEQ ID NO: 49; and/or a light chain variable region as shown in SEQ ID NO: 50 or having an amino acid sequence having at least 90%, 95%, 96%, 97%, 98%, or 99% homology to SEQ ID NO: 50.

6. An isolated humanized antibody binding to Siglec-15 or an antigen binding fragment thereof, comprising a heavy chain variable region of a sequence shown in SEQ ID NO: 41 with one or more mutations introduced at a position selected from a group consisting of M3, K19, S40, E42, R44, P61, T65, S77, T78, S84, S88, M93, V97, P117 and/or S120, and/or a light chain variable region of a sequence shown in SEQ ID NO: 42 with one or more mutations introduced at a position selected from a group consisting of V3, L4, A9, A12, V13, L15, Q17, A19, S22, K24, D32, Q46, P47, E59, I62, A64, N78, H80, P81, V82, E83, E84, A87, A104 and/or L110;
preferably, the humanized antibody or an antigen binding fragment thereof comprises a heavy chain variable region of a sequence shown in SEQ ID NO: 41 with one or more mutations introduced at a position selected from a group consisting of M3Q, K19R, S40A, E42G, R44G, P61A, T65K, S77N, T78S, S84N, S88A, M93V, V97A, P117Q and/or S120T, and/or a light chain variable region of a sequence shown in SEQ ID NO: 42 with one or more mutations introduced at a position selected from a group consisting of V3Q, L4M, A9S, A12S, V13A, L15V, Q17D, A19V, S22T, K24R, D32E, Q46K, P47A, E59Q, I62V, A64S, N78T, H80S, P81S, V82L, E83Q, E84P, A87F, A104Q and/or L110I;
more preferably, the humanized antibody or an antigen binding fragment thereof comprises a heavy chain variable region selected from a group consisting of SEQ ID NOs: 51, 53, 55, and 57, and/or a light chain variable region selected from a group consisting of SEQ ID NOs: 52, 54, 56, 58, and 82;
even more preferably,
the heavy chain variable region is shown in SEQ ID NO: 51, and/or the light chain variable region is shown in SEQ ID NO: 52; or
the heavy chain variable region is shown in SEQ ID NO: 53, and/or the light chain variable region is shown in SEQ ID NO: 54; or
the heavy chain variable region is shown in SEQ ID NO: 53, and/or the light chain variable region is shown in SEQ ID NO: 56; or
the heavy chain variable region is shown in SEQ ID NO: 53, and/or the light chain variable region is shown in SEQ ID NO: 58; or
the heavy chain variable region is shown in SEQ ID NO: 53, and/or the light chain variable region is shown in SEQ ID NO: 82; or
the heavy chain variable region is shown in SEQ ID NO: 55, and/or the light chain variable region is shown in SEQ ID NO: 56; or
the heavy chain variable region is shown in SEQ ID NO: 57, and/or the light chain variable region is shown in SEQ ID NO: 58.

7. An isolated humanized antibody binding to Siglec-15 or an antigen binding fragment thereof, comprising a heavy chain variable region of a sequence shown in SEQ ID NO: 45 with one or more mutations introduced at a position selected from a group consisting of E1, Q5, L11, V12, R13, L20, T23, K38, R40, T41, E42, A61, P62, K67, A68, A72, S76, N77, L81, H82, T85, T87, V97, and/or S113, and/or a light chain variable region of a sequence shown in SEQ ID NO: 46 with one or more mutations introduced at a position selected from a group consisting of S8, F12, A18, Y36, L41, P43, K45, V47, T58, D74, G78, N81, I82, P84, and/or V89;
preferably, the isolated humanized antibody binding to Siglec-15 or an antigen binding fragment thereof comprises a heavy chain variable region of a sequence shown in SEQ ID NO: 45 with one or more mutations introduced at a position selected from a group consisting of E1Q, Q5V, L11V, V12K, R13K, L20V, T23K, K38R, R40A, T41P, E42G, A61S, P62Q, K67R, A68V, A72R, S76T or S76A, N77S, L81M, H82E, T85S, T87R, V97A, and/or S113T, and/or a light chain variable region of a sequence shown in SEQ ID NO: 46 with one or more mutations introduced at a position selected from a group consisting of S8P, F12A, A18V, Y36H, L41E, P43G, K45R, V47L, T58K, D74E, G78T, N81S, I82L, P84S, and/or V89D;
more preferably, the isolated humanized antibody binding to Siglec-15 or an antigen binding fragment thereof comprises a heavy chain variable region selected from a group consisting of SEQ ID NOs: 59, 61, 63, and 65, and/or a light chain variable region selected from a group consisting of SEQ ID NOs: 60, 62, 64, and 66;
further preferably, the heavy chain variable region is shown in SEQ ID NO: 59, and/or the light chain variable region is shown in SEQ ID NO: 60; or
the heavy chain variable region is shown in SEQ ID NO: 61, and/or the light chain variable region is shown in SEQ ID NO: 62; or
the heavy chain variable region is shown in SEQ ID NO: 63, and/or the light chain variable region is shown in SEQ ID NO: 64.

8. An isolated humanized antibody binding to Siglec-15 or an antigen binding fragment thereof, comprising a heavy chain variable region of a sequence shown in SEQ ID NO: 47 with one or more mutations introduced at a position selected from a group consisting of E1, Q5, P9, V10, L11, V12, M20, E23, K38, S40, K43, S44, N61, K67, A68, L70, V72, S76, N84, T87, S91, and/or S119, and/or a light chain variable region of a sequence shown in SEQ ID NO: 48 with one or more mutations introduced at a position selected from a group consisting of V3, L4, A9, A12, V13, L15, Q17, A19, S22, R43, R46, P47, E59, I62, A64, R72, D80, P81, V82, E83, A84, D85, V87, A104, and/or L110;
preferably, the humanized antibody or an antigen binding fragment thereof comprises a heavy chain variable region of a sequence shown in SEQ ID NO: 47 with one or more mutations introduced at a position selected from a group consisting of E1Q, Q5V, P9A, V10E, L11V, V12K, M20V, E23K, K38R, S40A, K43Q, S44G, N61A, K67R, A68V, L70M, V72R, S76T, N84S, T87R, S91T, and/or S119T, and/or a light chain variable region of a sequence shown in SEQ ID NO: 48 with one or more mutations introduced at a position selected from a group consisting of V3Q, L4M, A9S, A12S, V13A, L15V, Q17D, A19V, S22T, R43K, R46K, P47A, E59Q, I62V, A64S, R72G, D80S, P81S, V82L, E83Q, A84P, D85E, V87F, A104Q, and/or L110I;
more preferably, the humanized antibody or an antigen binding fragment thereof comprises a heavy chain variable region selected from a group consisting of SEQ ID NOs: 67, 69, 71, and 73, and/or a light chain variable region selected from a group consisting of SEQ ID NOs: 68, 70, 72, and 74;
further more preferably,
the heavy chain variable region is shown in SEQ ID NO: 67, and/or the light chain variable region is shown in SEQ ID NO: 68; or
the heavy chain variable region is shown in SEQ ID NO: 69, and/or the light chain variable region is shown in SEQ ID NO: 70; or
the heavy chain variable region is shown in SEQ ID NO: 71, and/or the light chain variable region is shown in SEQ ID NO: 72.

9. The isolated antibody binding to Siglec-15 or an antigen binding fragment thereof, according to any one of claims 1 to 8, further comprising a constant region or a variant thereof, preferably a IgG1 or IgG4 constant region or a variant each thereof.

10. A nucleic acid sequence encoding the isolated antibody binding to Siglec-15 or an antigen binding fragment thereof according to any one of claims 1 to 9.

11. An expression vector comprising the nucleic acid sequence according to claim 10.

12. A host cell comprising the expression vector according to claim 11.

13. A method for preparing the isolated antibody binding to Siglec-15 or an antigen binding fragment thereof according to any one of claims 1 to 9, comprising
(a) culturing a host cell containing a nucleic acid encoding the antibody or an antigen binding fragment thereof in a culture medium in a condition enabling the expression of the nucleic acid, thereby producing a polypeptide comprising a light chain variable region and a heavy chain variable region; and
(b) recovering the polypeptide from the host cell or the culture medium.

14. A composition comprising the isolated antibody binding to Siglec-15 or an antigen binding fragment thereof according to any one of claims 1 to 9, and a pharmaceutically acceptable carrier or diluent.

15. An immune conjugate obtained by linking the isolated antibody binding to Siglec-15 or an antigen binding fragment thereof according to any one of claims 1 to 9, to a therapeutic agent.

16. A bispecific antibody comprising any of the isolated antibody binding to Siglec-15 or an antigen binding fragment thereof according to any one of claims 1 to 9.

17. Use of the isolated antibody binding to Siglec-15 or an antigen binding fragment thereof according to any one of claims 1 to 9, the composition according to claim 14, the immune conjugate according to claim 15, the bispecific antibody according to claim 16 in the preparation of a medicament for treatment of a Siglec-15 relevant disease;
preferably, the Siglec-15 relevant disease includes, but is not limited to, a cancer or a bone disease.

18. Use of the isolated antibody binding to Siglec-15 or an antigen binding fragment thereof according to any one of claims 1 to 9, the composition according to claim 14, the immune conjugate according to claim 15, the bispecific antibody according to claim 16 in the preparation of a medicament for inhibiting tumor cell growth.
